(19) Europäisches Patentamt
European Patent Office
Office européen des brevets

(11) EP 1 577 387 A1

(12) # EUROPEAN PATENT APPLICATION

published in accordance with Art. 158(3) EPC

(43) Date of publication:
**21.09.2005 Bulletin 2005/38**

(21) Application number: **03768133.5**

(22) Date of filing: **22.12.2003**

(51) Int Cl.7: **C12N 15/11**, C12Q 1/68, A61K 39/395, A61K 48/00, A61P 1/00, A61P 11/00, A61P 15/00, A61P 35/00, G01N 33/15, G01N 33/50, G01N 33/574

(86) International application number:
**PCT/JP2003/016417**

(87) International publication number:
**WO 2004/058969 (15.07.2004 Gazette 2004/29)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **24.12.2002 JP 2002373144**

(71) Applicant: **Takeda Pharmaceutical Company Limited**
**Osaka 541-0045 (JP)**

(72) Inventors:
- **HIKICHI, Yuichi**
**Tsukuba-shi, Ibaraki 305-0035 (JP)**
- **NISHIZAWA, Satoru**
**Tsukuba-shi, Ibaraki 305-0035 (JP)**

(74) Representative: **Lewin, John Harvey**
**Takeda Euro IP Department,**
**11-12 Charles II Street**
**London SW1Y 4QU (GB)**

(54) **PREVENTIVES/REMEDIES FOR CANCER**

(57) A compound or its salt that regulates (preferably inhibits) the activity of a protein comprising the same or substantially the same amino acid sequence as represented by SEQ ID NO: 1, a compound or its salt that regulates (preferably inhibits) the expression of a gene for the protein, an antisense polynucleotide comprising the entire or part of a base sequence complementary or substantially complementary to a base sequence of a polynucleotide encoding the protein or its partial peptide, an antibody against the protein, etc. can be used as a prophylactic/therapeutic agent for cancer, etc., an apoptosis inducing agent, or the like.



Printed by Jouve, 75001 PARIS (FR)

SEQUENCE LISTING
<110> Takeda Chemical Industries, Ltd.

<120> Preventing and treating agent for cancer

<130> 3130WOOP

<150> JP2002-373144
<151> 2002-12-24

<160> 14

<210> 1
<211> 751
<212> PRT
<213> Homo sapiens

<400> 1

```
Met Gly Gln Thr Gly Lys Lys Ser Glu Lys Gly Pro Val Cys Trp Arg
                5                   10                  15
Lys Arg Val Lys Ser Glu Tyr Met Arg Leu Arg Gln Leu Lys Arg Phe
            20                  25                  30
Arg Arg Ala Asp Glu Val Lys Ser Met Phe Ser Ser Asn Arg Gln Lys
        35                  40                  45
Ile Leu Glu Arg Thr Glu Ile Leu Asn Gln Glu Trp Lys Gln Arg Arg
    50                  55                  60
Ile Gln Pro Val His Ile Leu Thr Ser Val Ser Ser Leu Arg Gly Thr
65                  70                  75                  80
Arg Glu Cys Ser Val Thr Ser Asp Leu Asp Phe Pro Thr Gln Val Ile
                85                  90                  95
Pro Leu Lys Thr Leu Asn Ala Val Ala Ser Val Pro Ile Met Tyr Ser
            100                 105                 110
Trp Ser Pro Leu Gln Gln Asn Phe Met Val Glu Asp Glu Thr Val Leu
        115                 120                 125
His Asn Ile Pro Tyr Met Gly Asp Glu Val Leu Asp Gln Asp Gly Thr
    130                 135                 140
Phe Ile Glu Glu Leu Ile Lys Asn Tyr Asp Gly Lys Val His Gly Asp
145                 150                 155                 160
Arg Glu Cys Gly Phe Ile Asn Asp Glu Ile Phe Val Glu Leu Val Asn
                165                 170                 175
Ala Leu Gly Gln Tyr Asn Asp Asp Asp Asp Asp Asp Asp Gly Asp Asp
            180                 185                 190
Pro Glu Glu Arg Glu Glu Lys Gln Lys Asp Leu Glu Asp His Arg Asp
        195                 200                 205
Asp Lys Glu Ser Arg Pro Pro Arg Lys Phe Pro Ser Asp Lys Ile Phe
    210                 215                 220
Glu Ala Ile Ser Ser Met Phe Pro Asp Lys Gly Thr Ala Glu Glu Leu
225                 230                 235                 240
Lys Glu Lys Tyr Lys Glu Leu Thr Glu Gln Gln Leu Pro Gly Ala Leu
                245                 250                 255
Pro Pro Glu Cys Thr Pro Asn Ile Asp Gly Pro Asn Ala Lys Ser Val
            260                 265                 270
Gln Arg Glu Gln Ser Leu His Ser Phe His Thr Leu Phe Cys Arg Arg
        275                 280                 285
Cys Phe Lys Tyr Asp Cys Phe Leu His Arg Lys Cys Asn Tyr Ser Phe
    290                 295                 300
His Ala Thr Pro Asn Thr Tyr Lys Arg Lys Asn Thr Glu Thr Ala Leu
305                 310                 315                 320
Asp Asn Lys Pro Cys Gly Pro Gln Cys Tyr Gln His Leu Glu Gly Ala
                325                 330                 335
Lys Glu Phe Ala Ala Ala Leu Thr Ala Glu Arg Ile Lys Thr Pro Pro
            340                 345                 350
Lys Arg Pro Gly Gly Arg Arg Arg Gly Arg Leu Pro Asn Asn Ser Ser
        355                 360                 365
Arg Pro Ser Thr Pro Thr Ile Asn Val Leu Glu Ser Lys Asp Thr Asp
    370                 375                 380
Ser Asp Arg Glu Ala Gly Thr Glu Thr Gly Gly Glu Asn Asn Asp Lys
385                 390                 395                 400
```

```
Glu Glu Glu Glu Lys Lys Asp Glu Thr Ser Ser Ser Ser Glu Ala Asn
                405                 410                 415
Ser Arg Cys Gln Thr Pro Ile Lys Met Lys Pro Asn Ile Glu Pro Pro
            420                 425                 430
Glu Asn Val Glu Trp Ser Gly Ala Glu Ala Ser Met Phe Arg Val Leu
        435                 440                 445
Ile Gly Thr Tyr Tyr Asp Asn Phe Cys Ala Ile Ala Arg Leu Ile Gly
    450                 455                 460
Thr Lys Thr Cys Arg Gln Val Tyr Glu Phe Arg Val Lys Glu Ser Ser
465                 470                 475                 480
Ile Ile Ala Pro Ala Pro Ala Glu Asp Val Asp Thr Pro Pro Arg Lys
                485                 490                 495
Lys Lys Arg Lys His Arg Leu Trp Ala Ala His Cys Arg Lys Ile Gln
            500                 505                 510
Leu Lys Lys Asp Gly Ser Ser Asn His Val Tyr Asn Tyr Gln Pro Cys
        515                 520                 525
Asp His Pro Arg Gln Pro Cys Asp Ser Ser Cys Pro Cys Val Ile Ala
    530                 535                 540
Gln Asn Phe Cys Glu Lys Phe Cys Gln Cys Ser Ser Glu Cys Gln Asn
545                 550                 555                 560
Arg Phe Pro Gly Cys Arg Cys Lys Ala Gln Cys Asn Thr Lys Gln Cys
                565                 570                 575
Pro Cys Tyr Leu Ala Val Arg Glu Cys Asp Pro Asp Leu Cys Leu Thr
            580                 585                 590
Cys Gly Ala Ala Asp His Trp Asp Ser Lys Asn Val Ser Cys Lys Asn
        595                 600                 605
Cys Ser Ile Gln Arg Gly Ser Lys Lys His Leu Leu Leu Ala Pro Ser
    610                 615                 620
Asp Val Ala Gly Trp Gly Ile Phe Ile Lys Asp Pro Val Gln Lys Asn
625                 630                 635                 640
Glu Phe Ile Ser Glu Tyr Cys Gly Glu Ile Ile Ser Gln Asp Glu Ala
                645                 650                 655
Asp Arg Arg Gly Lys Val Tyr Asp Lys Tyr Met Cys Ser Phe Leu Phe
            660                 665                 670
Asn Leu Asn Asn Asp Phe Val Val Asp Ala Thr Arg Lys Gly Asn Lys
        675                 680                 685
Ile Arg Phe Ala Asn His Ser Val Asn Pro Asn Cys Tyr Ala Lys Val
    690                 695                 700
Met Met Val Asn Gly Asp His Arg Ile Gly Ile Phe Ala Lys Arg Ala
705                 710                 715                 720
Ile Gln Thr Gly Glu Glu Leu Phe Phe Asp Tyr Arg Tyr Ser Gln Ala
                725                 730                 735
Asp Ala Leu Lys Tyr Val Gly Ile Glu Arg Glu Met Glu Ile Pro
            740                 745                 750

<210> 2
<211> 2253
<212> DNA
<213> Homo sapiens

<400> 2
atgggccaga ctgggaagaa atctgagaag ggaccagttt gttggcggaa gcgtgtaaaa     60
tcagagtaca tgcgactgag acagctcaag aggttcagac gagctgatga agtaaagagt    120
atgtttagtt ccaatcgtca gaaaattttg gaaagaacgg aaatcttaaa ccaagaatgg    180
aaacagcgaa ggatacagcc tgtgcacatc ctgacttctg tgagctcatt gcgcgggact    240
agggagtgtt cggtgaccag tgacttggat tttccaacac aagtcatccc attaaagact    300
ctgaatgcag ttgcttcagt acccataatg tattcttggt ctcccctaca gcagaatttt    360
atggtggaag atgaaactgt tttacataac attccttata tgggagatga agttttagat    420
caggatggta ctttcattga agaactaata aaaaattatg atgggaaagt acacggggat    480
agagaatgtg ggtttataaa tgatgaaatt tttgtggagt tggtgaatgc ccttggtcaa    540
tataatgatg atgacgatga tgatgatgga gacgatcctg aagaaagaga agaaaagcag    600
aaagatctgg aggatcaccg agatgataaa gaaagccgcc cacctcggaa atttccttct    660
gataaaattt ttgaagccat ttcctcaatg tttccagata agggcacagc agaagaacta    720
aaggaaaaat ataaagaact caccgaacag cagctcccag gcgcacttcc tcctgaatgt    780
acccccaaca tagatggacc aaatgctaaa tctgttcaga gagagcaaag cttacactcc    840
tttcatacgc ttttctgtag gcgatgtttt aaatatgact gcttcctaca tcgtaagtgc    900
aattattctt ttcatgcaac acccaacact tataagcgga agaacacaga aacagctcta    960
gacaacaaac cttgtggacc acagtgttac cagcatttgg agggagcaaa ggagtttgct   1020
```

```
gctgctctca ccgctgagcg gataaagacc ccaccaaaac gtccaggagg ccgcagaaga  1080
ggacggcttc ccaataacag tagcaggccc agcaccccca ccattaatgt gctggaatca  1140
aaggatacag acagtgatag ggaagcaggg actgaaacgg ggggagagaa caatgataaa  1200
gaagaagaag agaagaaaga tgaaacttcg agctcctctg aagcaaattc tcggtgtcaa  1260
acaccaataa agatgaagcc aaatattgaa cctcctgaga atgtggagtg gagtggtgct  1320
gaagcctcaa tgtttagagt cctcattggc acttactatg acaatttctg tgccattgct  1380
aggttaattg ggaccaaaac atgtagacag gtgtatgagt ttagagtcaa agaatctagc  1440
atcatagctc cagctcccgc tgaggatgtg gatactcctc caaggaaaaa gaagaggaaa  1500
caccggttgt gggctgcaca ctgcagaaag atacagctga aaaaggacgg ctcctctaac  1560
catgtttaca actatcaacc ctgtgatcat ccacggcagc cttgtgacag ttcgtgccct  1620
tgtgtgatag cacaaaattt ttgtgaaaag ttttgtcaat gtagttcaga gtgtcaaaac  1680
cgctttccgg gatgccgctg caaagcacag tgcaacacca agcagtgccc gtgctacctg  1740
gctgtccgag agtgtgaccc tgacctctgt cttacttgtg gagccgctga ccattgggac  1800
agtaaaaatg tgtcctgcaa gaactgcagt attcagcggg gctccaaaaa gcatctattg  1860
ctggcaccat ctgacgtggc aggctggggg atttttatca aagatcctgt gcagaaaaat  1920
gaattcatct cagaatactg tggagagatt atttctcaag atgaagctga cagaagaggg  1980
aaagtgtatg ataaatacat gtgcagcttt ctgttcaact tgaacaatga ttttgtggtg  2040
gatgcaaccc gcaagggtaa caaaattcgt tttgcaaatc attcggtaaa tccaaactgc  2100
tatgcaaaag ttatgatggt taacggtgat cacaggatag gtatttttgc caagagagcc  2160
atccagactg gcgaagagct gtttttttgat tacagataca gccaggctga tgccctgaag  2220
tatgtcggca tcgaaagaga aatggaaatc cct                              2253
```

<210> 3
<211> 2695
<212> DNA
<213> Homo sapiens

<400> 3

```
caaataaaag cgatggcgat tgggctgccg cgtttggcgc tcggtccggt cgcgtccgac    60
acccggtggg actcagaagg cagtggagcc ccggcggcgg cggcggcggc gcgcgggggc   120
gacgcgcggg aacaacgcga gtcggcgcgc gggacgaaga ataatcatgg gccagactgg   180
gaagaaatct gagaagggac cagtttgttg gcggaagcgt gtaaaatcag agtacatgcg   240
actgagacag ctcaagaggt tcagacgagc tgatgaagta aagagtatgt ttagttccaa   300
tcgtcagaaa attttggaaa gaacggaaat cttaaaccaa gaatggaaac agcgaaggat   360
acagcctgtg cacatcctga cttctgtgag ctcattgcgc gggactaggg agtgttcggt   420
gaccagtgac ttggattttc caacacaagt catcccatta aagactctga atgcagttgc   480
ttcagtaccc ataatgtatt cttggtctcc cctacagcag aattttatgg tggaagatga   540
aactgtttta cataacattc cttatatggg agatgaagtt ttagatcagg atggtacttt   600
cattgaagaa ctaataaaaa attatgatgg gaaagtacac ggggatagag aatgtgggtt   660
tataaatgat gaaatttttg tggagttggt gaatgccctt ggtcaatata atgatgatga   720
cgatgatgat gatggagacg atcctgaaga aagagaagaa aagcagaaag atctggagga   780
tcaccgagat gataaagaaa gccgcccacc tcggaaattt ccttctgata aaatttttga   840
agccatttcc tcaatgtttc cagataaggg cacagcagaa gaactaaagg aaaaatataa   900
agaactcacc gaacagcagc tcccaggcgc acttcctcct gaatgtaccc ccaacataga   960
tggaccaaat gctaaatctg ttcagagaga gcaaagctta cactcctttc atacgctttt  1020
ctgtaggcga tgttttaaat atgactgctt cctacatcgt aagtgcaatt attcttttca  1080
tgcaacaccc aacacttata agcggaagaa cacagaaaca gctctagaca acaaaccttg  1140
tggaccacag tgttaccagc atttggaggg agcaaaggag tttgctgctg ctctcaccgc  1200
tgagcggata aagaccccac caaaacgtcc aggaggccgc agaagaggac ggcttcccaa  1260
taacagtagc aggcccagca cccccaccat taatgtgctg gaatcaaagg atacagacag  1320
tgatagggaa gcagggactg aaacgggggg agagaacaat gataaagaag aagaagagaa  1380
gaaagatgaa acttcgagct cctctgaagc aaattctcgg tgtcaaacac caataaagat  1440
gaagccaaat attgaacctc ctgagaatgt ggagtggagt ggtgctgaag cctcaatgtt  1500
tagagtcctc attggcactt actatgacaa tttctgtgcc attgctaggt taattgggac  1560
caaaacatgt agacaggtgt atgagtttag agtcaaagaa tctagcatca tagctccagc  1620
tcccgctgag gatgtggata ctcctccaag gaaaaagaag aggaaacacc ggttgtgggc  1680
tgcacactgc agaaagatac agctgaaaaa ggacggctcc tctaaccatg tttacaacta  1740
tcaaccctgt gatcatccac ggcagccttg tgacagttcg tgcccttgtg tgatagcaca  1800
aaatttttgt gaaaagtttt gtcaatgtag ttcagagtgt caaaaccgct ttccgggatg  1860
ccgctgcaaa gcacagtgca acaccaagca gtgcccgtgc tacctggctg tccgagagtg  1920
tgaccctgac ctctgtctta cttgtggagc cgctgaccat tgggacagta aaaatgtgtc  1980
ctgcaagaac tgcagtattc agcggggctc caaaaagcat ctattgctgg caccatctga  2040
cgtggcaggc tgggggattt ttatcaaaga tcctgtgcag aaaaatgaat tcatctcaga  2100
atactgtgga gagattattt ctcaagatga agctgacaga agagggaaag tgtatgataa  2160
atacatgtgc agctttctgt tcaacttgaa caatgatttt gtggtggatg caacccgcaa  2220
gggtaacaaa attcgttttg caaatcattc ggtaaatcca aactgctatg caaaagttat  2280
gatggttaac ggtgatcaca ggataggtat ttttgccaag agagccatcc agactggcga  2340
agagctgttt tttgattaca gatacagcca ggctgatgcc ctgaagtatg tcggcatcga  2400
```

aagagaaatg gaaatccctt gacatctgct acctcctccc ccctcctctg aaacagctgc 2460
cttagcttca ggaacctcga gtactgtggg caatttagaa aaagaacatg cagtttgaaa 2520
ttctgaattt gcaaagtact gtaagaataa tttatagtaa tgagtttaaa aatcaacttt 2580
ttattgcctt ctcaccagct gcaaagtgtt ttgtaccagt gaatttttgc aataatgcag 2640
tatggtacat ttttcaactt tgaataaaga atacttgaac ttgtcaaaaa aaaaa 2695

<210> 4
<211> 19
<212> DNA
<213> Artificial Sequence

<220>
<223> Primer

<400> 4
gcgcgggacg aagaataat 19

<210> 5
<211> 21
<212> DNA
<213> Artificial Sequence

<220>
<223> Primer

<400> 5
ggggaggagg tagcagatgt c 21

<210> 6
<211> 18
<212> DNA
<213> Artificial Sequence

<220>
<223> Primer

<400> 6
caagcagtgc ccgtgcta 18

<210> 7
<211> 21
<212> DNA
<213> Artificial Sequence

<220>
<223> Primer

<400> 7
agcggctcca caagtaagac a 21

<210> 8
<211> 25
<212> DNA
<213> Artificial Sequence

<220>
<223> Probe

<400> 8
tggctgtccg agagtgtgac cctga 25

<210> 9
<211> 20
<212> DNA
<213> Artificial Sequence

<220>
<223> oligonucleotide

<400> 9
aaacccacat tctctatccc 20

<210> 10
<211> 20
<212> DNA
<213> Artificial Sequence

<220>
<223> oligonucleotide

<400> 10
ccctatctct tacacccaaa 20

<210> 11
<211> 21
<212> DNA
<213> Artificial

<220>
<223> DNA/RNA molecule used as a siRNA

<400> 11
aaguugaaca gaaagcugct t 21

<210> 12
<211> 21
<212> DNA
<213> Artificial

<220>
<223> DNA/RNA molecule used as a siRNA

<400> 12
gcagcuuucu guucaacuut t 21

<210> 13
<211> 21
<212> DNA
<213> Artificial

<220>
<223> DNA/RNA molecule used as a siRNA

<400> 13
uucuccgaac gugucacgut t 21

<210> 14
<211> 21
<212> DNA
<213> Artificial

<220>
<223> DNA/RNA molecule used as a siRNA

<400> 14
acgugacacg uucggagaat t 21

## Description

TECHNICAL FIELD

The present invention relates to prophylactic/therapeutic agents and diagnostics for cancer, screening of the prophylactic/therapeutic agents for cancer, apoptosis inducing agents, screening of the apoptosis inducing agents, etc.

BACKGROUND ART

In cancer chemotherapy, the development of new anticancer drugs results in improved life-extending effects to increase cases moving toward cure. However, almost all anticancer drugs currently used cause damages on DNAs and exert potent cytotoxicity to arrest cell division. For these reasons, anticancer drugs considerably injure normal cells and serious side-effects often appear especially on the bone marrow with vigorous cell division.

To exhaustively analyze gene expression, a microarray analysis using immobilized cDNAs or oligonucleotides was developed so that techniques of detecting changes in disease-specific gene expression have come into wide use and its benefits have been established. For example, the GeneChip system of Affymetrix Corp. is going to be frequently used for diagnosis of diseases such as cancer, etc. and discovery of target genes for drug development.

Antisense oligonucleotides, when transfected to cells, hybridize to RNA having complementary sequence and induce degradation of RNAs by RNase H, inhibiting protein translation or causing inhibition of direct protein synthesis by hybridization. Since it is possible to specifically prevent functions of the objective gene, antisense oligonucleotides are widely used as a means for analyzing gene functions and in some of them, development is advancing toward clinical applications.

In recent years it gradually became clear that chromatin structure is deeply involved in regulating the division and growth of cells or the transcription of genes. In histones which constitute chromatin, it is known that especially the domains called histone tails undergo modifications such as acetylation, phosphorylation or methylation to contribute to change in the structure of chromatin (JIKKEN IGAKU, edited by Nakatani et al., October 2001).

The *Drosophila* enhancer of zeste is one of the members called the Polycomb group, which is a protein known to maintain homeotic gene repression by regulating chromatin.

To isolate genes associated with Down syndrome, Chen et al. performed exon trapping from the chromosome 21 cosmid library (Genomics, 38, 30-37, 1996). One of the exons that were cloned showed strong homology to the Drosophila enhancer of zeste protein. Chen et al. cloned the full-length gene and termed ENHANCER OF ZESTE, DROSOPHILA, HOMOLOG 2 (hereinafter sometimes abbreviated as EZH2) (Genomics, 38, 30-37, 1996). Cardoso et al. mapped EZH2 on chromosome and reported that EZH2 was present on 7q35, and at the same time, suggested that EZH2 gene is involved in the pathogenesis of malignant myeloid disorders, since this region is a region where aberrations are found in myeloid leukemia (Europ. J. Hum. Genet., 8, 174-180, 2000). Varambally et al. found that EZH2 is overexpressed in hormone-refractory prostate cancer and suggested a possibility that EZH2 might take part in malignant alteration of prostate cancer. Furthermore, they designed siRNA against EZH2 and reported that siRNA inhibited proliferation of the prostate cancer cell line, when introduced into cells (Nature, 419, 624-629, 2002). However, nothing other cancer species than prostate cancer is reported on the overexpression of EZH2. In addition, it is reported that siRNA described above did not induce apoptosis in cancer cells (Nature, 419, 624-629, 2002).

Cao et al. reported that they prepared a complex of a protein termed FED and EZH2 and this complex specifically methylates the 27-lysine residue of nucleosomal histone H3 (Science, 298, 1039-1043, 2002). In recent years, attention has been brought to histone protein modification, especially the relationship of histone protein deacetylation to cancers, and development of anti-cancer agents inhibiting the function of histone deacetylase as a target are under way. However, any anti-cancer agent targeting a histone methyltransferase has not been reported so far.

A drug targeting a molecule specifically expressed in cancer cells and capable of inhibiting cancer cell growth or inducing apoptosis has been earnestly desired.

DISCLOSRUE OF THE INVENTION

In order to solve the problems described above, the present inventors made extensive studies and have found that EZH2 is overexpressed in breast cancer, colon cancer, lung cancer, ovary cancer, pancreatic cancer, etc. and when an antisense oligonucleotide to EZH2 is introduced into cancer cells, the cancer cells cause apoptosis. The present invention has thus been accomplished.

That is, the present invention provides the following features, and so on.

(1) A prophylactic/therapeutic agent for cancer, comprising a compound or its salt that inhibits the activity of a protein comprising the same or substantially the same amino acid sequence as the amino acid sequence represented by SEQ ID NO: 1, its partial peptide, or a salt thereof.

(2) A prophylactic/therapeutic agent for cancer, comprising a compound or its salt that inhibits the expression of a gene for a protein comprising the same or substantially the same amino acid sequence as the amino acid sequence represented by SEQ ID NO: 1, its partial peptide, or a salt thereof.

(3) An antisense polynucleotide comprising the entire or part of a base sequence complementary or substantially complementary to a base sequence of a polynucleotide encoding a protein comprising the same or substantially the same amino acid sequence as the amino acid sequence represented by SEQ ID NO: 1, or its partial peptide.

(4) A prophylactic/therapeutic agent for cancer, comprising the antisense polynucleotide according to (3).

(5) A prophylactic/therapeutic agent for cancer, comprising an antibody against a protein comprising the same or substantially the same amino acid sequence as the amino acid sequence represented by SEQ ID NO: 1, its partial peptide, or a salt thereof.

(6) The prophylactic/therapeutic agent for cancer according to (1), (2), (4) or (5), wherein said cancer is colon cancer, breast cancer, lung cancer, pancreatic cancer or ovary cancer.

(6a) The prophylactic/therapeutic agent for cancer according to (1), (2), (4) or (5), wherein said cancer is a hormone-independent cancer.

(6b) The prophylactic/therapeutic agent for cancer according to (1), (2), (4) or (5), wherein said cancer is colon cancer, breast cancer, lung cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor or blood tumor.

(6c) The prophylactic/therapeutic agent for cancer according to (1), (2), (4) or (5), wherein said cancer is colon cancer, lung cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor or blood tumor.

(7) A diagnostic agent for cancer comprising an antibody against a protein comprising the same or substantially the same amino acid sequence as the amino acid sequence represented by SEQ ID NO: 1, its partial peptide, or a salt thereof.

(8) A diagnostic agent for cancer comprising a polynucleotide encoding a protein comprising the same or substantially the same amino acid sequence as the amino acid sequence represented by SEQ ID NO: 1, or its partial peptide.

(9) The diagnostic agent according to (7) or (8), wherein said cancer is colon cancer, breast cancer, lung cancer, pancreatic cancer or ovary cancer.

(9a) The diagnostic agent according to (7) or (8), wherein said cancer is a hormone-independent cancer.

(10) A prophylactic/therapeutic agent for a compound or its salt having an action of inhibiting histone methyltransferase activity.

(11) An apoptosis inducing agent comprising a compound or its salt having an action of inhibiting histone methyltransferase activity.

(12) A prophylactic/therapeutic agent for cancer comprising a compound or its salt having an action of inhibiting expression of histone methyltransferase .

(13) An apoptosis inducing agent comprising a compound or its salt having an action of inhibiting histone expression of methyltransferase .

(13a) The agent according to (10) to (13), wherein the histone methyltransferase is an enzyme to transfer the methyl group(s) to the lysine 9 and/or 27 residue of histone H3.

(14) A method of screening a prophylactic/therapeutic agent for cancer, which comprises using a protein comprising the same or substantially the same amino acid sequence as the amino acid sequence represented by SEQ ID NO: 1, its partial peptide, or a salt thereof.

(15) A kit for screening a prophylactic/therapeutic agent for cancer, comprising a protein comprising the same or substantially the same amino acid sequence as the amino acid sequence represented by SEQ ID NO: 1, its partial peptide, or a salt thereof.

(16) A method of screening a prophylactic/therapeutic agent for cancer, which comprises using a polynucleotide encoding a protein comprising the same or substantially the same amino acid sequence as the amino acid sequence represented by SEQ ID NO: 1, or its partial peptide.

(17) A kit for screening a prophylactic/therapeutic agent for cancer, comprising a polynucleotide encoding a protein comprising the same or substantially the same amino acid sequence as the amino acid sequence represented by SEQ ID NO: 1, or its partial peptide.

(17a) A prophylactic/therapeutic agent for cancer, which is obtainable by using the screening method according to (14) or (16), or the screening kit according to (15) or (17).

(18) An apoptosis inducing agent comprising a compound or its salt that inhibits the activity of a protein comprising the same or substantially the same amino acid sequence as the amino acid sequence represented by SEQ ID NO:

1, its partial peptide, or a salt thereof.

(19) An apoptosis inducing agent comprising a compound or its salt that inhibits the expression of a gene for a protein comprising the same or substantially the same amino acid sequence as the amino acid sequence represented by SEQ ID NO: 1, its partial peptide, or a salt thereof.

(20) A method of screening an apoptosis inducing agent, which comprises using a protein comprising the same or substantially the same amino acid sequence as the amino acid sequence represented by SEQ ID NO: 1, its partial peptide, or a salt thereof.

(21) A method of screening an apoptosis inducing agent, which comprises using a polynucleotide encoding a protein comprising the same or substantially the same amino acid sequence as the amino acid sequence represented by SEQ ID NO: 1, or its partial peptide.

(21a) An apoptosis inducing agent, which is obtainable by using the screening method according to (20) or (21).

(22) A method of preventing/treating cancer, which comprises administering to a mammal an effective dose of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, its partial peptide or a salt thereof, (iii) an antibody against said protein, its partial peptide or a salt thereof, or (iv) an antisense polynucleotide comprising the entire or part of a base sequence complementary or substantially complementary to a base sequence of a polynucleotide encoding said protein or its partial peptide.

(23) A method of inducing apoptosis, which comprises administering to a mammal an effective dose of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, its partial peptide or a salt thereof, (iii) an antibody against said protein, its partial peptide or a salt thereof, or (iv) an antisense polynucleotide comprising the entire or part of a base sequence complementary or substantially complementary to a base sequence of a polynucleotide encoding said protein or its partial peptide.

(24) A method of preventing/treating cancer, which comprises inhibiting the activity of a protein comprising the same or substantially the same amino acid sequence as the amino acid sequence represented by SEQ ID NO: 1, its partial peptide, or a salt thereof, or inhibiting the expression of a gene for said protein, its partial peptide, or a salt thereof.

(25) A method of inducing apoptosis, which comprises inhibiting the activity of a protein comprising the same or substantially the same amino acid sequence as the amino acid sequence represented by SEQ ID NO: 1, its partial peptide, or a salt thereof, or inhibiting the expression of a gene for said protein, its partial peptide, or a salt thereof.

(26) Use of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, its partial peptide or a salt thereof, (iii) an antibody against said protein, its partial peptide or a salt thereof, or (iv) an antisense polynucleotide comprising the entire or part of a base sequence complementary or substantially complementary to a base sequence of a polynucleotide encoding said protein or its partial peptide, to manufacture a prophylactic/therapeutic agent for cancer.

(27) Use of (i) a compound or its salt that inhibits the activity of a protein comprising the same or substantially the same amino acid sequence as the amino acid sequence represented by SEQ ID NO: 1, its partial peptide, or a salt thereof, (ii) a compound or its salt that inhibits the expression of a gene for said protein, its partial peptide or a salt thereof, (iii) an antibody against said protein, its partial peptide or a salt thereof, or (iv) an antisense polynucleotide comprising the entire or part of a base sequence complementary or substantially complementary to a base sequence of a polynucleotide encoding said protein or its partial peptide, to manufacture an apoptosis inducing agent.

## BEST MODE FOR CARRYING OUT THE INVENTION

The protein, which has the same or substantially the same amino acid sequence as the amino acid sequence represented by SEQ ID NO: 1 (hereinafter the protein is sometimes referred to as the protein of the present invention or the protein used in the present invention) may be any protein derived from any cells of human and warm-blooded animals (e.g., guinea pig, rat, mouse, fowl, rabbit, swine, ovine, bovine, simian, etc.) (such as hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e. g., large intestine and small intestine), blood vessel, heart, thymus, spleen, submandibular gland, peripheral blood, prostate, testis, ovary, placenta, uterus, bone, joint, skeletal muscle, etc.; the protein may also be a synthetic protein.

The amino acid sequence having substantially the same amino acid sequence as that represented by SEQ ID NO: 1 includes amino acid sequences having at least about 50% homology, preferably at least about 60% homology, more preferably at least about 70% homology, much more preferably at least about 80% homology, further much more preferably at least about 90% homology and most preferably at least about 95% homology, to the amino acid sequence shown by SEQ ID NO: 1; and so on.

Homology of the amino acid sequences can be determined under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

Preferred examples of the protein comprising substantially the same amino acid sequence as the amino acid sequence represented by SEQ ID NO: 1 include proteins having substantially the same amino acid sequence as the amino acid sequence represented by SEQ ID NO: 1 and having an activity of substantially the same property as that of the protein comprising the amino acid sequence represented by SEQ ID NO: 1, and the like.

The activity of substantially the same property includes, for example, a histone methyltransferase (e.g., an enzyme to transfer the methyl group(s) to the lysine 9 and/or 27 residue of histone H3, etc.) activity, and the like. The "substantially the same property" is used to mean that the property of these activities is equivalent in terms of quality (e.g., physiologically or pharmacologically). Thus, the activities described above are preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The histone methyltransferase activity can be assayed by publicly known methods, e.g., the method described in *Science,* 298, 1039-1043, 2002, or with its modifications. In more detail, (i) the protein of the present invention-EED complex, (ii) S-adenosyl-L-methionine with a radiolabeled methyl group and (iii) histone protein, oligonucleosome or a polypeptide having a sequence around lysine 27 of histone H3 are reacted and the radioactivity of the polypeptide or histone H3 by transmethylation is determined. The reaction is carried out in an appropriate buffer. After the enzyme reaction, the reaction product is separated by, e.g., SDS-PAGE, etc. and first, compared to the mobility of histone H3, etc. as a standard control for identification. In quantitative determination, the radioactivity is measured by publicly known methods using a scintillation counter, fluorography, etc.

Examples of the protein used in the present invention include so-called muteins such as proteins comprising (i) the amino acid sequence represented by SEQ ID NO: 1, of which at least 1 or 2 (e.g., about 1 to about 100, preferably about 1 to about 30, more preferably about 1 to about 10 and most preferably several (1 to 5)) amino acids are deleted; (ii) the amino acid sequence represented by SEQ ID NO: 1, to which at least 1 or 2 (e.g., about 1 to about 100, preferably about 1 to about 30, more preferably about 1 to about 10 and most preferably several (1 to 5)) amino acids are added; (iii) the amino acid sequence represented by SEQ ID NO: 1, in which at least 1 or 2 (e.g., about 1 to about 100, preferably about 1 to about 30, more preferably about 1 to about 10 and most preferably several (1 to 5)) amino acids are inserted; (iv) the amino acid sequence represented by SEQ ID NO: 1, in which at least 1 or 2 (e.g., about 1 to about 100, preferably about 1 to about 30, more preferably about 1 to about 10 and most preferably several (1 to 5)) amino acids are substituted by other amino acids; or (v) a combination of these amino acid sequences; and the like.

Where the amino acid sequence is inserted, deleted or substituted as described above, the position of its insertion, deletion or substitution is not particularly limited.

Throughout the specification, the proteins are represented in accordance with the conventional way of describing proteins, that is, the N-terminus (amino terminus) at the left hand and the C-terminus (carboxyl terminus) at the right hand. In the protein used in the present invention including the protein comprising the amino acid sequence represented by SEQ ID NO: 1, the C-terminus may be in any form of a carboxyl group (-COOH), a carboxylate (-COO-), an amide ($-CONH_2$) and an ester (-COOR).

Herein, examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl such as a phenyl-$C_{1-2}$ alkyl group, e.g., benzyl, phenethyl, etc.; an α-naphthyl-$C_{1-2}$ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include those in which the amino group at the N-terminal amino acid residues (e.g., methionine residue) is protected with a protecting group (e.g., a $C_{1-6}$ acyl

group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group, e.g., formyl group, acetyl group, etc.), or conjugated proteins such as glycoproteins having sugar chains; etc.

Specific examples of the protein used in the present invention are a protein (EZH2) comprising the amino acid sequence represented by SEQ ID NO: 1, and the like.

The partial peptide of the protein used in the present invention may be any peptide as long as it is a partial peptide of the protein used in the present invention described above and preferably has the property equivalent to that of the protein used in the present invention described above.

For the purpose of preparing the antibody of the present invention later described, specific examples of the peptide include peptides having the 1st to 610th amino acid sequence in the amino acid sequence represented by SEQ ID NO: 1. Preferably used are peptides having, e.g., at least 20, preferably at least 50, more preferably at least 70, much more preferably at least 100, and most preferably at least 200, amino acids in the constituent amino acid sequence of the protein used in the present invention, and the like.

The partial peptide used in the present invention may contain deletion of at least 1 or 2 (preferably about 1 to about 20, more preferably about 1 to about 10 and most preferably several (1 to 5)) amino acids in the amino acid sequence; addition of at least 1 or 2 (preferably about 1 to about 20, more preferably about 1 to about 10 and most preferably several (1 to 5)) amino acids in the amino acid sequence; insertion of at least 1 or 2 (preferably about 1 to about 20, more preferably about 1 to about 10 and most preferably several (1 to 5)) amino acids in the amino acid sequence; or substitution of at least 1 or 2 (preferably about 1 to about 20, more preferably about 1 to 10 and most preferably several (1 to 5)) amino acids in the amino acid sequence by other amino acids.

In the partial peptide used in the present invention, the C-terminus may be in any form of a carboxyl group (-COOH), a carboxylate (-COO-), an amide ($-CONH_2$) or an ester (-COOR).

Furthermore, the partial peptide used in the present invention includes those having a carboxyl group (or a carboxylate) at a position other than the C-terminus, those having an amino group protected with a protecting group at the N-terminal amino acid residues (e.g., methionine residue); those being cleaved at the N-terminal region in vivo and with the glutamyl group thus formed being pyroglutaminated; those having a substituent on the side chain of an amino acid in the molecule wherein the substituent is protected with a suitable protecting group, or conjugated peptides such as so-called glycopeptides having sugar chains; etc., as in the protein used in the present invention described above.

The partial peptide used in the present invention may also be used as an antigen for producing antibodies.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by publicly known methods used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be later described.

Where these proteins are manufactured from human or mammalian tissues or cells, human or mammalian tissues or cells are homogenized, extracted with an acid or the like, and the extract is purified/isolated by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2', 4'-dimethoxyphenyl-hydroxymethyl)phenoxy resin, 4-(2',
4'-dimethoxyphenyl-Fmoc-aminoethyl) phenoxy resin, etc. Using these resins, amino acids, in which $\alpha$-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N, N'

-diisopropylcarbodiimide, N-ethyl-N'-(3-dimethylaminopropyl) carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents that are known to be usable for protein condensation reactions. Examples of such solvents are acid amides such as N, N-dimethylformamide, N, N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower ($C_{1-6}$) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2, 3, 6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2, 4, 5-trichlorophenol, 2, 4-dinitrophenol, cyanomethyl alcohol, p-nitrophenol, HONB, N-hydroxysuccimide, N-hydroxyphthalimide, HOBt)], etc. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1, 4-butanedithiol, 1, 2-ethanedithiol, etc. Furthermore, 2, 4-dinitrophenyl group known as the protecting group for the imidazole of histidine is removed by a treatment with thiophenol. Formyl group used as the protecting group of the indole of tryptophan is eliminated by the aforesaid acid treatment in the presence of 1, 2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Then, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the $\alpha$-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. The methods for peptide synthesis include, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (i) to (v) below.

(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method or its modification; when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

The DNA encoding the protein used in the present invention may be any one of, for example, a DNA comprising the base sequence represented by SEQ ID NO: 2, or a DNA comprising a base sequence hybridizable to the base sequence represented by SEQ ID NO: 2 under high stringent conditions and encoding a protein which has the properties of substantially the same property as those of the protein having the amino acid sequence represented by SEQ ID NO: 1 described above. The DNA comprising the base sequence represented by SEQ ID NO: 2 includes a DNA comprising the base sequence represented by SEQ ID NO: 3, etc.

Specific examples of the DNA that is hybridizable to the base sequence represented by SEQ ID NO: 2 under high stringent conditions include DNAs comprising at least about 50% homology, preferably at least about 60% homology, more preferably at least about 70% homology, much more preferably at least about 80% homology, further much more preferably at least about 90% homology and most preferably at least about 95% homology, to the base sequence represented by SEQ ID NO: 2; and the like.

Homology in the base sequence can be measured under the following conditions (an expectation value = 10; gaps are allowed; filtering = ON; match score = 1; mismatch score = -3) using the homology scoring algorithm NCBI BLAST (National Center for Biotechnology Information Basic Local Alignment Search Tool).

The hybridization can be carried out by publicly known methods or by modifications thereof, for example, by the method described in Molecular Cloning, 2nd (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, as the DNA encoding the protein comprising the amino acid sequence represented by SEQ ID NO: 1, etc., there are employed a DNA containing the base sequence represented by SEQ ID NO: 2, etc.

The DNA encoding the partial peptide used in the present invention may be any DNA so long as it contains the base sequence encoding the partial peptide used in the present invention described above. The DNA may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA comprising a part of the DNA having the base sequence represented by SEQ ID NO: 2, or a DNA comprising a base sequence hybridizable to the base sequence represented by SEQ ID NO: 2 under high stringent conditions and comprising a part of DNA encoding a protein having the activities of substantially the same property as those of the protein of the present invention, and the like.

The DNA hybridizable to the base sequence represented by SEQ ID NO: 2 indicates the same meaning as described above.

Methods for the hybridization and the high stringent conditions that can be used are the same as those described above.

For cloning of the DNA that completely encodes the protein or partial peptide used in the present invention (hereinafter sometimes merely referred to as the protein of the present invention in the description of cloning of DNAs encoding the protein and partial peptide and their expression), the DNA can be either amplified by PCR using synthetic DNA primers containing a part of the base sequence of the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method, the Kunkel method, etc., or its modification, using PCR, a publicly known kit available as Mutan$^{TM}$-super Express Km (manufactured by Takara Shuzo Co., Ltd.) or Mutan$^{TM}$-K (manufactured by Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form Escherichia coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, $\lambda P_L$ promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin promoter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are genus Escherichia, genus Bacillus, yeast, insect cells, insects, animal cells, etc.

Specific examples of the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R$^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr$^-$) cell), mouse L cell, mouse AtT-20, mouse myeloma cell, mouse ATDC5 cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55 (1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus Escherichia or the genus Bacillus, the transformant can be appropriately cultured in a liquid medium, which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature), 195, 788 (1962)) to which an appropriate additive such as immobilized 10% bovine serum is added. Preferably, pH of the medium is adjusted to about 6.2 to 6.4. Normally, the transformant is cultivated at about 27°C for about 3 to 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30 to 40°C for about 15 to 60 hours and, if necessary, the culture can be aerated or agitated.

As described above, the protein of the present invention can be produced in the cell of, in the cell membrane of, or outside of the transformant.

The protein of the present invention can be separated and purified from the culture described above by the following procedures.

When the protein of the present invention is extracted from the culture of bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100™, etc. When the protein of the present invention is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies against the protein or partial peptide used in the present invention, or its salts may be any of polyclonal and monoclonal antibodies, as long as they are capable of recognizing the protein or partial peptide used in the present invention, or its salts.

The antibodies against the protein or partial peptide used in the present invention, or its salts (hereinafter they are sometimes collectively referred to as the protein of the present invention in the description of the antibodies) can be produced by a publicly known method of producing an antibody or antiserum, using the protein of the present invention as an antigen.

[Preparation of monoclonal antibody]

(a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are simian, rabbits, canine, guinea pigs, mice, rats, ovine, goats and fowl, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g.; a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally under 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

[Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and the animal is immunized with the complex in a manner similar to the method described above for the manufacture of monoclonal antibodies. The product containing the antibody against the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described above.

The antisense polynucleotide having a complementary or substantially complementary base sequence to the base sequence of a polynucleotide encoding the protein or partial peptide used in the present invention (e.g., DNA (hereinafter these DNAs are sometimes collectively referred to as the DNA of the present invention in the description of antisense polynucleotide)) can be any antisense polynucleotide, so long as it possesses a base sequence complementary or substantially complementary to the base sequence of the polynucleotide (e.g., DNA) of the present invention and capable of suppressing the expression of said DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. Especially in the entire base sequence of the complementary strand to the DNA of the present invention, preferred are (a) an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon) in the case of antisense polynucleotide directed to translation inhibition and (b) an antisense polynucleotide having at least about 70% homology, preferably at least about 80% homology, more preferably at least about 90% homology and most preferably at least about 95% homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to a base sequence of DNA containing the base sequence represented by SEQ ID NO: 2 or SEQ ID NO: 3, preferably an antisense polynucleotide containing the entire or part of a base sequence complementary to a base sequence of DNA containing the base sequence represented by SEQ ID NO: 2 or SEQ ID NO: 3 (more preferably, an antisense polynucleotide containing the entire or part of a base sequence complementary to a base sequence of DNA containing the base sequence represented by SEQ ID NO: 2 or SEQ ID NO: 3), etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 2. These antisense polynucleotides may be synthesized using a publicly known DNA synthesizer, etc.

According to the present invention, the antisense polynucleotide (nucleic acid) capable of inhibiting the replication or expression of a gene for the protein of the present invention can be designed and synthesized based on the base sequence information of cloned or identified protein-encoding DNA. Such an antisense polynucleotide is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating and/or controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and proteins usually refer to amino acids of a protein under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense." Examples of the antisense polynucleotides include polynucleotides containing 2-deoxy-D-ribose, polynucleotides containing D-ribose, any other type of polynucleotides which are N-glycosides of a purine or pyrimidine base, or other polymers having non-nucleotide backbones (e.g., commercially available protein nucleic acids and synthetic sequence-specific nucleic acid polymers) or other polymers containing particular linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid are sulfur and thiophosphate derivatives of nucleic acids, those resistant to degradation of polynucleoside amides or oligonucleoside amides, etc. The antisense polynucleotide of the present invention can be modified preferably based on the following design, that is, by increasing the intracellular stability of the antisense polynucleotide, enhancing the cell permeability of the antisense polynucleotide, increasing the affinity of the polynucleotide to the targeted sense strand to a higher level, or minimizing the toxicity, if any, of the antisense polynucleotide. Many of such modifications are known in the art, as disclosed in J. Kawakami, et al., Pharm. Tech. Japan, Vol. 8, pp. 247, 1992; Vol. 8, pp. 395, 1992; Antisense Research and Applications, CRC Press, 1993; etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory activity of the antisense polynucleotide can be examined using the transformant of the present invention, the gene expression system of the present invention in vivo and in vitro, or the translation system for the protein of the present invention in vivo and in vitro.

Hereinafter, the protein of the present invention, its partial peptides, or salts thereof (hereinafter sometimes merely referred to as the protein of the present invention), the polynucleotide (e.g., DNA) (hereinafter sometimes merely referred to as the DNA of the present invention) encoding the protein of the present invention or its partial peptides, the antibodies against the protein of the present invention, its partial peptides, or salts thereof (hereinafter sometimes referred to as the antibodies of the present invention) and the antisense polynucleotides to the polynucleotide (e.g., DNA) of the present invention (hereinafter sometimes merely referred to as the antisense polynucleotides of the present invention) are specifically described for their applications.

The protein of the present invention is increasingly expressed in cancer tissues and hence, can be used as a marker for disease. That is, the protein of the present invention is useful as a marker for the early diagnosis in cancer tissues, judgment of severity in conditions, or predicted development of diseases. The pharmaceutical comprising the antisense polynucleotide of a gene encoding the protein of the present invention, the compound or its salt that inhibits the activity of the protein of the present invention or the antibody against the protein of the present invention can be used as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, a prophylactic/therapeutic agent for hormone-dependent cancer, an apoptosis inducing agent, etc.

(1) Screening of pharmaceutical candidate compounds for disease

The protein of the present invention is increasingly expressed in cancer tissues and has an apoptosis suppressing effect. Also, the antisense polynucleotide of the present invention has the cell death effect of cancer cells, the effect of inducing or promoting apoptosis, etc. Thus, the compound or its salt that regulates (promotes or inhibits, preferably inhibits) the activity of the protein of the present invention can be used as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer. In addition, the compound or its salt can be used as an apoptosis inducing agent. Alternatively, the compound or its salt can be used as a prophylactic/therapeutic agent for hormone-dependent cancer.

Accordingly, the protein of the present invention is useful as a reagent for screening the compound or its salt that regulates (inhibits or promotes, preferably inhibits) the activity of the protein of the present invention.

That is, the present invention provides a method of screening the compound or its salt that regulates (inhibits or promotes, preferably inhibits) the activity (e.g., the histone methyltransferase activity, etc.) of the protein of the present invention.

More specifically, there is employed a method of screening the compound or its salt that regulates (inhibits or promotes, preferably inhibits) the activity of the protein of the present invention, which comprises comparing, e.g., (i) the histone methyltransferase activity of the protein of the present invention, with (ii) the histone methyltransferase activity of a mixture of the protein of the present invention and a test compound.

In the screening method described above, the histone methyltransferase activity is assayed, for example, by known methods, e.g., the method described in *Science,* 298, 1039-1043, 2002, or its modifications, followed by comparison, in the cases of (i) and (ii).

Specifically, the compound or its salt that regulates (inhibits or promotes, preferably inhibits) the activity of the protein of the present invention is screened by measuring radioactivities of histone H3 or the polypeptide by transmethylation, respectively, (i) in the case where (a) the protein of the present invention-EED complex, (b) S-adenosyl-L-methionine with a radiolabeled methyl group and (c) histone protein, oligonucleosome or a polypeptide having a sequence around lysine 27 of histone H3 are reacted and (ii) in the case where (a) the protein of the present invention-EED complex, (b) S-adenosyl-L-methionine with a radiolabeled methyl group and (c) histone protein, oligonucleosome or a polypeptide having a sequence around lysine 27 of histone H3 are reacted in the presence of a test compound.

The reaction is carried out in an appropriate buffer. After the enzyme reaction, the reaction product is separated by, e.g., SDS-PAGE, etc. and first, compared to the mobility of histone H3, etc. as a standard control for identification. In quantitative determination, the radioactivity is measured by publicly known methods using a scintillation counter, fluorography, etc.

The S-adenosyl-L-methionine with a radiolabeled methyl group and histone protein, oligonucleosome or the polypeptide having a sequence around lysine 27 of histone H3 may be mixed with a test compound, and then reacted with the protein of the present invention-EED complex. Alternatively, the S-adenosyl-L-methionine with a radiolabeled methyl group and histone protein, oligonucleosome or the polypeptide having a sequence around lysine 27 of histone H3 may be brought in contact with the present invention-EED complex, followed by adding a test compound to the mixture.

Also, the compound or its salt that regulates (inhibits or promotes, preferably inhibits) the activity of the protein of the present invention is screened by measuring methylated (monomethylated, dimethylated and/or trimethylated) lysine residues using, e.g., anti-histone H3 (dimethyl/trimethyl lysine 27) antibody, etc., respectively, (i') in the case where (a) the protein of the present invention-EED complex, (b) S-adenosyl-L-methionine and (c) histone protein, oligonucleosome or a polypeptide having a sequence around lysine 27 of histone H3 are reacted and (ii') in the case where (a) the protein of the present invention-EED complex, (b) S-adenosyl-L-methionine with a radiolabeled methyl group and (c) histone protein, oligonucleosome or a polypeptide having a sequence around lysine 27 of histone H3 are reacted in the presence of a test compound.

Furthermore, the compound or its salt that regulates (inhibits or promotes, preferably inhibits) the activity of the protein of the present invention is screened by measuring changes in molecular weight of the respective reaction products (products purified depending on necessity) accompanied by methylation using mass spectrometry (using e.

g., TOF-MS, etc.), (i") in the case where (a) the protein of the present invention-EED complex, (b) S-adenosyl-L-methionine and (c) histone protein, oligonucleosome or a polypeptide having a sequence around lysine 27 of histone H3 are reacted and (ii") in the case where (a) the protein of the present invention-EED complex, (b) S-adenosyl-L-methionine and (c) histone protein, oligonucleosome or a polypeptide having a sequence around lysine 27 of histone H3 are reacted in the presence of a test compound.

Preferably, the protein of the present invention described above is the one produced by culturing transformants containing DNA encoding the protein of the present invention. Furthermore, the reaction is similarly performed using cells capable of producing the protein of the present invention and the radioactivity of histone H3 or the polypeptide by transmethylation can be determined.

As the cells capable of producing the protein of the present invention, there are used, for example, a host (transformant) transformed with a vector containing the DNA encoding the protein of the present invention. Preferably, animal cells such as COS7 cells, CHO cells, HEK293 cells, etc. are used as the host. For the screening, the transformant, in which the protein of the present invention has been secreted extracellularly or expressed in the cells, e.g., by culturing through the procedures described above, is preferably employed. The procedures for culturing the cells capable of expressing the protein of the present invention are similar to the culturing procedures for the transformant of the present invention described above.

Examples of the test compound include peptides, proteins, non-peptide compounds, synthetic compounds, fermentation products, cell extracts, plant extracts, animal tissue extracts, etc.

For example, when a test compound decreases the histone methyltransferase activity in the case of (ii) described above by at least about 20%, preferably at least 30% and more preferably at least about 50%, as compared to the case of (i) described above, the test compound can be selected as the compound capable of inhibiting the activity of the protein of the present invention; when a test compound increases the above activity in the case of (ii) described above by at least about 20%, preferably at least 30% and more preferably at least about 50%, as compared to the case of (i) described above, the test compound can be selected as the compound capable of promoting the activity of the protein of the present invention.

The compound having the activity of inhibiting the activity of the protein of the present invention is useful as a safe and low toxic pharmaceutical to suppress the physiological activity of the protein of the present invention, such as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, an apoptosis inducing agent (for cancer cells), etc.

The compound having the activity of promoting the activity of the protein of the present invention is useful as a safe and low-toxic pharmaceutical to potentiate the effect of the protein of the present invention.

The compound or its salt obtained using the screening method or screening kit of the present invention is the compound selected from, for example, peptides, proteins, non-peptide compounds, synthetic compounds, fermentation products, cell extracts, plant extracts, animal tissue extracts, plasma, etc. The salts of these compounds used are those given above as the salts of the peptide of the present invention.

In addition, the gene encoding the protein of the present invention is also increasingly expressed in cancer cells. Also, the antisense polynucleotide of the present invention has the effect of cell death of cancer cells, the effect of inducing/promoting apoptosis, etc. Accordingly, the compound or its salt that regulates the expression of the gene encoding the protein of the present invention can be used as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, an apoptosis inducing agent, etc.

Therefore, the DNA of the present invention is useful as a reagent for screening the compound or its salt that regulates (inhibits or promotes, preferably inhibits) the expression of the gene encoding the protein of the present invention.

For the screening, there is a method of screening which comprises comparing (iii) the case where a cell capable of producing the protein of the present invention is cultured and (iv) the case where a cell capable of producing the protein used in the present invention is cultured in the presence of a test compound.

In the method described above, the expression level of the gene described above (specifically, the level of the protein of the present invention or the level of mRNA encoding the above protein) is determined in the cases of (iii) and (iv), followed by comparison.

Examples of the test compound and the cells capable of producing the protein of the present invention are the same as described above.

The level of the protein of the present invention can be determined by publicly known methods, e.g., by measuring the aforesaid protein present in the cell extract, etc., using an antibody capable of recognizing the said protein, in accordance with methods such as western blot analysis, ELISA, etc., or their modifications.

The mRNA level can be determined by publicly known methods, e.g., in accordance with methods such as Northern hybridization using a nucleic acid containing the entire or a part of SEQ ID NO: 2 as a probe, or PCR using a nucleic acid containing the entire or a part of SEQ ID NO: 2 as a primer, or modifications thereof.

For example, when a test compound inhibits the expression level of the gene in the case (iv) described above by at least about 20%, preferably at least 30% and more preferably at least about 50%, as compared to the case (iii) above, the test compound can be selected to be a compound capable of inhibiting the expression of the gene encoding the protein of the present invention; when a test compound increases the expression level of the gene in the case (iv) described above by at least about 20%, preferably at least 30% and more preferably at least about 50%, as compared to the case (iii) above, the test compound can be selected to be a compound capable of promoting the expression of the gene encoding the protein of the present invention.

The screening kit of the present invention comprises the protein used in the present invention, its partial peptide or salts thereof, or the cell capable of producing the protein used in the present invention, or its partial peptide.

The compound or its salts obtained by using the screening method or screening kit of the present invention is the test compound described above, e.g., a compound selected from peptides, proteins, non-peptide compounds, synthetic compounds, fermentation products, cell extracts, plant extracts, animal tissue extracts, plasma, etc., or its salt, which is a compound or its salts regulating (preferably inhibiting) the activity of the protein of the present invention (e.g., the histone methyltransferase activity, etc.) or the expression of the gene for the protein of the present invention.

The salts of these compounds used are those given above as the salts of the protein of the present invention.

The compound or its salts that regulate (preferably inhibit) the activity of the protein of the present invention or the compound or its salts that regulate (preferably inhibit) the expression of a gene encoding the protein of the present invention are low toxic and useful as prophylactic/therapeutic agents for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, or as prophylactic/therapeutic agents for hormone-independent cancer, an apoptosis inducing agent, etc.

Where the compound or its salts obtained by using the screening method or screening kit of the present invention are used as the prophylactic/therapeutic agents described above, these compounds can be converted into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a carrier, a diluent or excipient conventionally used in the field of pharmaceutical preparations. Examples of the carrier or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injection, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the aforesaid antibody is contained in about 5 to about 100 mg especially in the form of injection, and in 10 to 250 mg for the other forms.

Each composition described above may further contain other active components unless formulation causes any adverse interaction with the compound described above.

Since the pharmaceutical preparations thus obtained are safe and low toxic, they can be administered to human or warm-blooded animal (e.g., mouse, rat, rabbit, ovine, swine, bovine, equine, fowl, feline, canine, simian, chimpanzee, etc.) orally or parenterally.

The dose of the compound or its salts may vary depending upon its action, target disease, subject to be administered, route of administration, etc. For example, when the compound or its salt that regulates (preferably inhibits) the activity of the protein of the present invention is orally administered for the purpose of treating, e.g., breast cancer, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the said compound or its salt may vary depending upon subject to be administered, target disease, etc. When the compound or its salt that regulates (preferably inhibits) the activity of the protein of the present invention is administered to an adult (as 60 kg body weight) in the form of an injectable preparation for the purpose of treating, e.g., breast cancer, it is advantageous to administer the compound or its salt by way of intravenous injection in a daily dose of about 0.01 to about 30 mg, preferably about 0.1 to about 20 mg, and more preferably about 0.1 to about 10 mg. For other animal species, the corresponding dose as converted per 60 kg weight can be administered.

Also, the compounds described above can be used in combination with known anticancer agents [e.g., alkylating agents (e.g., cyclophosphamide, ifosfamide, nimustine, ranimustine, carboquone, etc.), metabolic antagonists (e.g., methotrexate, 5-fluorouracil, tegafur, carmofur, UFT, doxifluridine, cytarabine, enocitabine, mercaptopurine, mercaptopurine riboside, thioguanine, etc.), antitumor antibiotics (e.g., mitomycin, adriamycin, daunorubicin, epirubicin, pirarubicin, idarubicin, bleomycin, peplomycin, actinomycin, etc.), plant-derived antitumor agents (e.g., vincristine, vinblastine, vindesine, etoposide, camptothecin, Irinotecan, etc.), cisplatin, carboplatin, nedaplatin, paclitaxel, docetaxel, estramustine, etc.]. On such occasions, timing of administration is not limited; these agents may be administered to the target subject simultaneously or at staggered times. The dose may be appropriately chosen on the dose which is clinically applied. A ratio of the compound described above to the antitumor agent can be appropriately selected, depending on the subject to be administered, administration route, target disease, clinical conditions, combination, etc.

(2) Quantification for the protein of the present invention, its partial peptide or salts thereof

The antibody against the protein of the present invention (hereinafter sometimes merely referred to as the antibody of the present invention) is capable of specifically recognizing the protein of the present invention, and thus can be used for quantification of the protein of the present invention in a test sample fluid, in particular, for quantification by sandwich immunoassay; etc.

That is, the present invention provides:

(i) a method of quantifying the protein of the present invention in a test sample fluid, which comprises competitively reacting the antibody of the present invention, a test sample fluid and a labeled form of the protein of the present invention, and measuring the ratio of the labeled form of the protein of the present invention bound to said antibody; and,

(ii) a method of quantifying the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody against the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or $F(ab')_2$, Fab' or Fab fractions of the antibody molecule may also be used.

The method of quantifying the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, lanthanide, and the like. As the radioisotopes, there are used, e.g., [$^{125}I$], [$^{131}I$], [$^{3}H$], [$^{14}C$], etc. The enzymes described above are preferably enzymes, which are stable and have a high specific activity, and include, e.g., β-galactosidase, β-glucosidase, an alkaline phosphatase, a peroxidase, malate dehydrogenase, etc. As the fluorescent substances, there are used, e.g., cyanine fluorescent dyes (e.g., Cy2, Cy3, Cy5, Cy5.5, Cy7 (manufactured by Amersham Biosciences Corp.), etc.), fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method of the present invention, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody against the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody against the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying each of these immunological methods to the quantification method of the present invention, any particular conditions or procedures are not required. Quantification system for the protein of the present invention or its salts is constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the protein of the present invention can be quantified with high sensitivity, using the antibody of the present invention.

Furthermore, when an increased or decreased level of the protein of the present invention is detected by quantifying the level of the protein of the present invention using the antibody of the present invention, it can be diagnosed that one suffers from, for example, cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably, colon cancer, breast cancer, lung cancer, pancreatic cancer or ovary cancer; or it is highly likely to suffer from these disease in the future.

In addition, the antibody of the present invention can be used to detect the protein of the present invention, which is present in a test sample such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

(3) Gene diagnostic agent

By using the DNA of the present invention, e.g., as a probe, the DNA can detect an abnormality (gene abnormality) of the DNA or mRNA encoding the protein of the present invention or its partial peptide in human or warm-blooded animal (e.g., rat, mouse, guinea pig, rabbit, fowl, ovine, swine, bovine, equine, feline, canine, simian, chimpanzee, etc.). Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression or decreased expression is detected by, e.g., Northern hybridization or DNA mutation is detected by the PCR-SSCP assay, it can be diagnosed that it is highly likely to suffer from, for example, cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably, colon cancer, breast cancer, lung cancer, pancreatic cancer or ovary cancer.

(4) Pharmaceutical comprising the antisense polynucleotide

Since the antisense polynucleotide of the present invention that binds to the DNA of the present invention complementarily to suppress the expression of said DNA has the effect of cell death of cancer cells, the effect of inducing/promoting apoptosis, etc. and is low toxic, the antisense polynucleotide can suppress the function (e.g., histone methyltransferase activity) of the protein of the present invention or the DNA of the present invention in vivo. Thus, the antisense polynucleotide can be used, for example, as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, an apoptosis inducing agent, or the like.

Where the antisense polynucleotide described above is used as the aforesaid prophylactic/therapeutic agent, it can be prepared into pharmaceutical preparations by publicly known methods, which are provided for administration.

For example, when the antisense polynucleotide described above is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or mammal (e.g., rat, rabbit, ovine, swine, bovine, feline, canine, simian, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense polynucleotide may be prepared into an aerosol, which is topically administered into the trachea as an inhaler.

Further for the purposes of improving pharmacokinetics, extending a half-life and improving intracellular uptake efficiency, the antisense polynucleotide described above is prepared into pharmaceutical preparations (injectable preparations) alone or together with a carrier such as liposome, etc. and the preparations may be administered intravenously, subcutaneously, in articular cavities or at the affected area, etc.

A dose of the antisense polynucleotide may vary depending on target disease, subject to be administered, route for administration, etc. For example, where the antisense polynucleotide of the present invention is administered for the purpose of treating breast cancer, the antisense polynucleotide is generally administered to an adult (60 kg body weight) in a daily dose of about 0.1 to 100 mg.

Furthermore, the antisense polynucleotide may also be used as an oligonucleotide probe for diagnosis to examine the presence of the DNA of the present invention in tissues or cells and states of its expression.

As in the antisense polynucleotide described above, the double-stranded RNA containing a part of RNA encoding the protein of the present invention, ribozyme containing a part of RNA encoding the protein of the present invention, etc. can also prevent the expression of the gene encoding the present invention to suppress the in vivo function of the protein used in the present invention or the DNA used in the present invention and hence can be used, for example, as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, an apoptosis inducing agent, or the like.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes a portion proximal to a cleavage site on the RNA of the present invention, which may be cleaved by a publicly known ribozyme (RNA fragment).

Where the double-stranded RNA or ribozyme described above is used as the prophylactic/therapeutic agent described above, the double-stranded RNA or ribozyme is prepared into pharmaceutical preparations as in the antisense polynucleotide, and the preparations can be provided for administration.

(5) Pharmaceutical comprising the antibody of the present invention

The antibody of the present invention, which has the activity of neutralizing the activity of the protein of the present invention can be used, for example, as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, or an apoptosis inducing agent. The antibody can also be used as a prophylactic/therapeutic agent for a hormone-independent cancer.

The prophylactic/therapeutic agent comprising the antibody of the present invention for the diseases described above is low toxic and can be administered to human or mammals (e.g., rats, rabbits, ovine, swine, bovine, feline, canine, simian, etc.) orally or parenterally (e.g., intravenously) in the form of liquid preparation as it is or as a pharmaceutical composition of appropriate dosage form. The dose may vary depending upon subject to be administered, target disease, conditions, route of administration, etc. For example, when the agent is used for the purpose of treating, e.g., breast cancer in an adult, it is advantageous to administer the antibody of the present invention normally in a single dose of about 0.01 to about 20 mg/kg body weight, preferably about 0.1 to about 10 mg/kg body weight, and more preferably about 0.1 to about 5 mg/kg body weight, approximately 1 to 5 times per day, preferably approximately 1 to 3 times per day. In other parenteral administration and oral administration, the agent can be administered in a dose corresponding to the dose given above. When the condition is especially severe, the dose may be increased according to the condition.

The antibody of the present invention can be administered directly or as a pharmaceutical composition of appropriate dosage form. The pharmaceutical composition used for the administration comprises the antibody described above or its salt, pharmaceutically acceptable carriers, dilutes or excipients. Such a composition is provided as a dosage form appropriate for oral or parenteral (e.g., intravenous) administration, preferably, as an inhaler.

Each composition described above may further contain other active components, unless their formulation with the antibody causes any adverse interaction.

(6) "Prophylactic/therapeutic agent for cancer comprising the compound or its salt that has the activity of inhibiting the histone methyltransferase activity," "prophylactic/therapeutic agent for cancer comprising the compound or its salt that has the activity of inhibiting expression of a histone methyltransferase," "an apoptosis inducing agent comprising the compound or its salt that has the activity of inhibiting the histone methyltransferase activity" and "an apoptosis inducing agent comprising the compound or its salt that has the activity of inhibiting expression of a histone methyltransferase" of the present invention

The histone methyltransferase includes, for example, an enzyme to transfer the methyl group(s) to the lysine 9 and/or 27 residue of histone H3, etc.

The "compound that has the activity of inhibiting the histone methyltransferase activity" may be any compound, so long as it is a compound having the activity of inhibiting the histone methyltransferase activity. The compound is used, for example, as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, as an apoptosis inducing agent, etc.

The "compound that has the activity of inhibiting expression of the histone methyltransferase" may be any compound, so long as it is a compound having the activity of inhibiting expression of the histone methyltransferase. The compound is used, for example, as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, as an apoptosis inducing agent, etc.

These prophylactic/therapeutic agents and inducing agents are prepared as described above.

(7) DNA transgenic animal

The present invention provides a non-human mammal bearing DNA encoding the protein of the present invention, which is exogenous (hereinafter abbreviated as the exogenous DNA of the present invention) or its variant DNA (sometimes simply referred to as the exogenous variant DNA of the present invention).

That is, the present invention provides:

(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and to utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, ovine, goat, rabbits, canine, feline, guinea pigs, hamsters, mice, rats, etc. Above all, preferred are rodents, especially mice (e.g., C57B1/6 strain, DBA2 strain, etc. for a pure line and for a cross line, $B6C3F_1$ strain, $BDF_1$ strain $B6D2F_1$ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals, human, etc.

The exogenous DNA of the present invention refers to the DNA of the present invention that is once isolated/extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the protein of the present invention which is abnormal and exemplified by the DNA, etc. that expresses a protein for suppressing the function of the protein of the present invention which is normal.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, canine, feline, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, canine, feline, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of enhancing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, canine, feline, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygotic animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, a mammal transfected with the exogenous normal DNA of the present invention exhibits a symptom of increasing the protein of the present invention liberated. Thus, the animal is available for the screening test of, for example, prophylactic/therapeutic agents for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, or an apoptosis inducing agent.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention is overexpressed, and may eventually develop the function inactive type inadaptability to the protein of the present invention by inhibiting the function of endogenous normal DNA. Therefore, the animal can be utilized as a pathologic model animal for such a disease. For example, using the abnormal DNA transgenic animal of the present invention, it is possible to elucidate the pathological mechanism of the function inactive type inadaptability to the protein of the present invention and investigate how to treat this disease.

As a specific example of the availability, the transgenic animal overexpressing the abnormal DNA of the present invention is expected to serve as an experimental model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows a symptom of increasing the protein of the present invention liberated, the animal is also expected to serve for the screening test of prophylactic/therapeutic agents for the function inactive type inadaptability of the protein of the present invention, e.g., prophylactic/therapeutic agents for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), prophylactic/therapeutic agents for colon cancer, breast cancer, lung cancer, pancreatic cancer or ovary cancer, or apoptosis inducing agents.

Other potential applications of two kinds of the DNA transgenic animals of the present invention described above further include:

(i) Use as a cell source for tissue culture;

(ii) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;

(iii) Research on the function of cells derived from tissues that are usually cultured only with difficulty, using cells in tissues bearing the DNA cultured by a standard tissue culture technique;

(iv) Screening of an agent that enhances the function of cells using the cells described in (iii) above; and,
(v) Isolation and purification of the variant protein of the present invention and preparation of an antibody thereto; etc.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.
It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal can serve to identify cells capable of producing the protein of the present invention, and to study in association with apoptosis, differentiation or propagation or on the mechanism of signal transduction in these properties to inspect any abnormality therein. Accordingly, the DNA transgenic animal can provide an effective research material for the protein of the present invention and for investigation of the function and effect thereof.
To develop a therapeutic agent for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

(8) Knockout animal

The present invention provides a non-human mammal embryonic stem cell bearing the DNA of the present invention inactivated and a non-human mammal deficient in expressing the DNA of the present invention.
Thus, the present invention provides:

(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from Escherichia coli);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e.g., β-galactosidase derived from Escherichia coli) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).
As the non-human mammal, those described above are used.
Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.
Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the $BDF_1$ mouse ($F_1$ between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In addition thereto, embryos are preferably collected at the 8-cell stage, cultured until the blastocyte stage and then used thereby to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for cytological study of the protein of the present invention in vitro.

The non-human mammal deficient in expression of the DNA of the present invention can be identified from a normal animal by measuring the mRNA level in the subject animal by a publicly known method, and indirectly comparing the degrees of expression.

As the non-human mammal, those as given above are used.

With respect to the non-human mammal deficient in expression of the DNA of the present invention, the DNA of the present invention can be knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the protein of the present invention, such an animal can be a disease model suspected of inactivated biological activities of the protein of the present invention and thus, offers an effective study to investigate the causes for and therapy for these diseases.

(8a) Method of screening the compound having therapeutic/prophylactic effects on diseases caused by deficiency, damages, etc. of the DNA of the present invention

The non-human mammal deficient in expression of the DNA of the present invention can be employed for screening the compound having therapeutic/prophylactic effects on diseases caused by deficiency, damages, etc. of the DNA of the present invention.

That is, the present invention provides a method of screening a compound or its salt having an effect of treating/preventing a disease caused by deficiency, damages, etc. of the DNA of the present invention, e.g., cancer, etc., which comprises administering a test compound to a non-human mammal deficient in expression of the DNA of the present invention and observing/determining changes in the animal.

As the non-human mammal deficient in expression of the DNA of the present invention, which can be employed for the screening method, the same examples as described above apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration route, property of the test compound, etc.

When a compound having an effect of treating/preventing, for example, cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.) is screened, a test compound is administered to the non-human mammal deficient in expression of the DNA of the present invention. Differences in incidence of cancer or differences in degree of healing from the group administered with no test compound are observed in the tissues described above with passage of time.

In the screening method, when a test compound is administered to a test animal and the disease conditions of the test animal are improved by at least about 10%, preferably at least about 30% and more preferably at least about 50%, the test compound can be selected as the compound having therapeutic/prophylactic effects on the diseases described above.

The compound obtained using the above screening method is a compound selected from the test compounds described above and exhibits therapeutic/prophylactic effects on diseases caused by deficiencies, damages, etc. of the protein of the present invention. Therefore, the compound can be employed as a safe and low toxic prophylactic/therapeutic agents for these diseases. Furthermore, compounds derived from the compound obtained by the screening described above may also be used as well.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or mammal (e.g., rat, mouse, guinea pig, rabbit, ovine, swine, bovine, equine, feline, canine, simian, etc.).

The dose of the compound or its salt may vary depending upon target disease, subject to be administered, route of administration, etc. For example, when the compound is orally administered, the compound is administered to the adult patient with breast cancer (as 60 kg body weight) generally in a dose of about 0.1 to 100 mg, preferably about 1.0 to 50 mg and more preferably about 1.0 to 20 mg. In parenteral administration, a single dose of the compound may vary depending upon subject to be administered, target disease, etc. When the compound is administered to the adult patient with breast cancer (as 60 kg body weight) in the form of an injectable preparation, it is advantageous to administer the compound in a single dose of about 0.01 to about 30 mg, preferably about 0.1 to about 20 mg and more preferably about 0.1 to about 10 mg a day. For other animal species, the corresponding dose as converted per 60 kg weight can be administered.

(8b) Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The test compounds are those as given above.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from *Escherichia coli,* β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt promoting or inhibiting the promoter activity to the DNA of the present invention can regulate the expression of the protein of the present invention and can regulate the functions of the said protein. Thus, the compound or its salt is useful, for example, as a prophylactic/therapeutic agent for cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably as a prophylactic/therapeutic agent for colon cancer, breast cancer, lung cancer, pancreatic cancer or ovary cancer, as an apoptosis inducing agent, or the like.

In addition, compounds derived from the compound obtained by the screening described above may also be used as well.

A pharmaceutical comprising the compound obtained by the above screening method or a salt thereof can be manufactured in a manner similar to the method for preparing the pharmaceutical comprising the protein of the present invention described above.

Since the pharmaceutical preparation thus obtained is safe and low toxic, it can be administered to human or mammal (e.g., rat, mouse, guinea pig, rabbit, ovine, swine, bovine, equine, feline, canine, simian, etc.).

A dose of the compound or its salt may vary depending on target disease, subject to be administered, route for administration, etc.; when the compound that inhibits the promoter activity to the DNA of the present invention is orally administered, the compound is administered to the adult patient with breast cancer (as 60 kg body weight) normally in a daily dose of about 0.1 to 100 mg, preferably about 1.0 to 50 mg and more preferably about 1.0 to 20 mg. In parenteral administration, a single dose of the compound may vary depending on subject to be administered, target disease, etc. but when the compound of inhibiting the promoter activity to the DNA of the present invention is administered to the adult patient with breast cancer (as 60 kg body weight) in the form of injectable preparation, it is advantageous to administer the compound intravenously to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of prophylactic/therapeutic agents for these diseases.

In addition, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification, where bases, amino acids, etc. are expressed in abbreviations, they are denoted by abbreviations in accordance with the IUPAC-IUB Commission on Biochemical Nomenclature or by conventional abbreviations in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| A: | adenine |
| T: | thymine |
| G: | guanine |
| C: | cytosine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP: | adenosine triphosphate |
| EDTA : | ethylenediaminetetraacetic acid |
| SDS: | sodium dodecyl sulfate |
| Gly: | glycine |
| Ala: | alanine |
| Val: | valine |
| Leu: | leucine |
| Ile: | isoleucine |
| Ser: | serine |
| Thr: | threonine |
| Cys: | cysteine |
| Met: | methionine |
| Glu: | glutamic acid |
| Asp: | aspartic acid |
| Lys: | lysine |
| Arg: | arginine |
| His: | histidine |
| Phe: | phenylalanine |
| Tyr: | tyrosine |
| Trp: | tryptophan |
| Pro: | proline |
| Asn: | asparagine |
| Gln: | glutamine |
| pGlu: | pyroglutamic acid |
| Sec: | selenocysteine |

Substituents, protecting groups and reagents generally used in this specification are presented as the codes below.

| | |
|---|---|
| Me: | methyl group |
| Et: | ethyl group |
| Bu: | butyl group |
| Ph: | phenyl group |
| TC: | thiazolidine-4(R)-carboxamido group |
| Tos: | p-toluenesulfonyl |
| CHO : | formyl |
| Bzl: | benzyl |
| $Cl_2$-Bzl : | 2,6-dichlorobenzyl |
| Bom : | benzyloxymethyl |
| Z: | benzyloxycarbonyl |
| Cl-Z: | 2-chlorobenzyloxycarbonyl |
| Br-Z: | 2-bromobenzyl oxycarbonyl |
| Boc: | t-butoxycarbonyl |

DNP : dinitrophenol
Trt: trityl
Bum : t-butoxymethyl
Fmoc : N-9-fluorenyl methoxycarbonyl
HOBt: 1-hydroxybenztriazole
HOOBt : 3,4-dihydro-3-hydroxy-4-oxo-1,2,3-benzotriazine
HONB: 1-hydroxy-5-norbornene-2,3-dicarboxyimide
DCC : N,N'-dicyclohexylcarbodiimide

The sequence identification numbers in the sequence listing of the specification indicates the following sequence.

[SEQ ID NO: 1]
This shows the amino acid sequence of EZH2.
[SEQ ID NO: 2]
This shows the base sequence of DNA encoding EZH2.
[SEQ ID NO: 3]
This shows the base sequence of DNA containing the full-length gene encoding EZH2.
[SEQ ID NO: 4]
This shows the base sequence of the primer used in EXAMPLE 2.
[SEQ ID NO: 5]
This shows the base sequence of the primer used in EXAMPLE 2.
[SEQ ID NO: 6]
This shows the base sequence of the primer used in EXAMPLES 2, 4 and 5.
[SEQ ID NO: 7]
This shows the base sequence of the primer used in EXAMPLES 2, 4 and 5.
[SEQ ID NO: 8]
This shows the base sequence of the probe used in EXAMPLES 2, 4 and 5.
[SEQ ID NO: 9]
This shows the base sequence of the antisense oligonucleotide used in EXAMPLE 3.
[SEQ ID NO: 10]
This shows the base sequence of the antisense oligonucleotide for control used in EXAMPLE 3.
[SEQ ID NO: 11]
This shows the base sequence of the siRNA antisense strand of EZH2 used in EXAMPLE 5.
[SEQ ID NO: 12]
This shows the base sequence of the siRNA sense strand of EZH2 used in EXAMPLE 5.
[SEQ ID NO: 13]
This shows the base sequence of the siRNA used in EXAMPLE 5.
[SEQ ID NO: 14]
This shows the base sequence of the siRNA used in EXAMPLE 5.

Hereinafter the present invention will be described more specifically by referring to EXAMPLES but is not deemed to be limited thereto.

EXAMPLE 1

In order to clarify a group of genes overexpressed specifically in the breast cancer tissue, total RNAs as materials were extracted from 3 cases of breast normal tissue, 4 cases of breast cancer tissue, 2 cases of normal lung tissue, 2 cases of lung cancer tissue and other 23 types of normal tissue (Tables 1 and 2) and subjected to gene expression analysis using an oligonucleotide microarray (Human Genome U95A, U95B, U95C, U95D, U95E; Affymetrix Corp.).
The experiment was carried out in accordance with the protocol of Affymetrix Corp. (Expression analysis technical manual). As a result, overexpression of EZH2 was detected in breast cancer and lung cancer tissues. In the analyzed normal tissues other than rectum, cerebellum and testis, the expression was below the detection limit [scored as Absent by GeneChip analysis software (manufactured by Affymetrix)] (Tables 1 and 2).

Table 1

| RNA-Extracted Tissue | Distribution Source | Gene Expression Level |
|---|---|---|
| Breast normal tissue (Sample #1) | BioClinical Partners, Inc. | ND |
| Breast normal tissue (Sample #2) | BioClinical Partners, Inc. | ND |
| Breast normal tissue (Sample #15) | BioClinical Partners, Inc. | 0.176 |
| Breast cancer tissue (Patient #3) | BioClinical Partners, Inc. | 3.708 |
| Breast cancer tissue (Patient #4) | BioClinical Partners, Inc. | 0.398 |
| Breast cancer tissue (Patient #9) | BioClinical Partners, Inc. | 7.72 |
| Breast cancer tissue (Patient #11) | BioClinical Partners, Inc. | 1.403 |
| Lung normal tissue (Sample #10) | BioClinical Partners, Inc. | ND |
| Lung normal tissue (Sample #13) | BioClinical Partners, Inc. | ND |
| Lung cancer tissue (Patient #16) | BioClinical Partners, Inc. | 0.406 |
| Lung cancer tissue (Patient #20) | BioClinical Partners, Inc. | 0.445 |

The medial value for the expression level of all genes, which expression was detected by the oligonucleotide microarray, was taken as 1 to standardize the gene expression level.

ND: not detected

Table 2

| RNA-Extracted Tissue | Distribution Source | Gene Expression Level |
|---|---|---|
| Fat | BioChain Institute, Inc. | ND |
| Skeletal muscle | Clontech Laboratories, Inc. | ND |
| Heart | Clontech Laboratories, Inc. | ND |
| Kidney | Clontech Laboratories, Inc. | ND |
| Pancreas | Clontech Laboratories, Inc. | ND |
| Liver | Clontech Laboratories, Inc. | ND |
| Adrenal | Clontech Laboratories, Inc. | ND |
| Spleen | Clontech Laboratories, Inc. | ND |
| Trachea | Clontech Laboratories, Inc. | ND |
| Lung | Clontech Laboratories, Inc. | ND |
| Whole brain | Clontech Laboratories, Inc. | ND |
| Cerebellum | Clontech Laboratories, Inc. | 0.76 |
| Mammary gland | Clontech Laboratories, Inc. | ND |
| Salivary gland | Clontech Laboratories, Inc. | ND |
| Stomach | Clontech Laboratories, Inc. | ND |
| Rectum | BioChain Institute, Inc. | 0.745 |
| Large intestine | BioChain Institute, Inc. | ND |
| Small intestine | BioChain Institute, Inc. | ND |
| Uterus | Clontech Laboratories, Inc. | ND |
| Uterine cervix | BioChain Institute, Inc. | ND |
| Testis | Clontech Laboratories, Inc. | 12.85 |
| Prostate | Clontech Laboratories, Inc. | ND |

The medial value for the expression level of all genes, which expression was detected by the oligonucleotide microarray, was taken as 1 to standardize the gene expression level.

ND: not detected

EXAMPLE 2

(1) The full-length gene for EZH2 was cloned.

Using Marathon Ready cDNA library (manufactured by Clontech Laboratories, Inc.) as a template, PCR was carried out with two primers (SEQ ID NO: 4 and SEQ ID NO: 5), using Pfu polymerase (manufactured by Stratagene). After 20 µl of a reaction mixture containing 10 µl of 2 x GC buffer I (manufactured by Takara), 1.6 µl of 2.5 mM each dNTP mixture, 0.4 µl each of the above two primers, which were prepared to become 20 µM, 0.5 µl of a template cDNA solution and 0.4 µl of Pfu polymerase was pretreated at 96°C for 2 minutes, PCR was carried out by repeating 35 cycles set to include the reaction at 94°C for 10 seconds, 61 °C for 15 seconds and 72°C for 5 minutes as one cycle. After completion of the PCR, the product was separated by agarose gel electrophoresis and the desired band was cut out. The reaction product was purified using Gel Extraction Kit (manufactured by Qiagen). Subsequently, A was added to the reaction product at the both ends, based on the protocol attached to pcDNA3.1/V5 His TA Expression vector (manufactured by Invitrogen Corp.) to clone into the vector. The base sequence in the inserted fragment was confirmed thereby to verify that there was no error in the sequence, to obtain the EZH2-expressed vector (Vector 1).

(2) Using quantitative PCR, it was confirmed if EZH2 was overexpressed in various types of cancer.

Using two primes (SEQ ID NO: 6 and SEQ ID NO: 7) and TaqMan probe (SEQ ID NO: 8), quantitative PCR was carried out using Matched cDNA Pairs (manufactured by Clontech) as a template (Table 3). Using TaqMan Universal PCR Master Mix (manufactured by Applied Biosystems, Inc.), 15 µl of the reaction solution was prepared so that the final concentrations of primers, probe and template cDNA reached 500 nM, 100 nM and 1 µl, respectively. The reaction conditions were set to default conditions in ABI PRISM$^{TM}$ 7900HT Sequence Detector (manufactured by Applied Biosystems, Inc.). Vector 1 obtained in (1) above was provided for the PCR in a given number of copies to determine the copy number of EZH2 per 1 µl of the matched cDNA pair. A ratio of the expression level of EZH2 per 1 µl of cancer tissue-derived cDNA to the expression level of EZH2 per 1 µl of peripheral normal tissue-derived cDNA to form a pair was calculated. The results are shown in Table 3.

EZH2 was found to be overexpressed in 3 out of 4 cases with breast cancer, 2 out of 3 cases in colon cancer and 4 out of 4 cases with ovary cancer, as compared to their peripheral normal tissues.

Table 3

| Template | Ratio in Gene Expression Level |
|---|---|
| Human Breast 1 | 1.02 |
| Human Breast 2 | 4.19 |
| Human Breast 3 | 2.35 |
| Human Breast 4 | 7.65 |
| Human Rectum 1 | 9.92 |
| Human Rectum 2 | 0.49 |
| Human Rectum 3 | 5.47 |
| Human Ovary 1 | 33.2 |
| Human Ovary 3 | 4.59 |
| Human Ovary 4 | 7.30 |
| Human Ovary 5 | 12.8 |
| Human Prostate 1 | 13.7 |
| Human Prostate 3 | 0.76 |

In the table, the ratio of gene expression level represents the numerical value calculated by the following equation.

Ratio in gene expression level

= (number of EZH2 copies/µl of cancer tissue-derived cDNA)/(number of EZH2 copies/µl of peripheral normal tissue-derived cDNA)

EXAMPLE 3

In order to analyze the effects of EZH2 gene observed to be overexpressed in various types of cancer on apoptosis, the experiment of EZH2 antisense oligonucleotide transfection was performed.

First, an antisense (SEQ ID NO: 9) to the base sequence represented by SEQ ID NO: 3 was designed and then a phosphorothioated oligonucleotide was synthesized and purified on HPLC, which was provided for the transfection experiment (Amersham Pharmacia Biotech) (hereinafter briefly referred to as the antisense oligonucleotide). As a control oligonucleotide, reverse sequence (SEQ ID NO: 10) of the base sequence represented by SEQ ID NO: 9 was similarly phosphorothioated and purified on HPLC, and the phosphorothioated product was used (Amersham Pharmacia Biotech).

Breast cancer cell line MDA-MB-231 (In Vitro, 14 (11), 911-915, 1978, purchased from ATCC) was used as cells to be tested and on the preceding day of oligonucleotide transfection, 1 x $10^5$ cells were plated on a 24-well plate (manufactured by Falcon Co., Ltd.). Transfection of the oligonucleotide was performed using Lipofectamine 2000 (manufactured by Invitrogen Corp.) following instructions of the manufacturer. Fifteen hours after transfection, total RNA was extracted using the RNeasy Mini Kit (manufactured by Qiagen, Inc.) following the protocol of the manufacturer and cDNA was prepared using TaqMan Reverse Transcription Reagents (manufactured by Applied Biosystems, Inc.). Quantitative PCR was performed on ABI PRISM™ 7900HT Sequence Detector (manufactured by Applied Biosystems, Inc.), using two primers (SEQ ID NO: 6 and SEQ ID NO: 7) and a probe (SEQ ID NO: 8), as in EXAMPLE 2.

Meanwhile, the effect on apoptosis was assessed as follows. On the preceding day of oligonucleotide transfection, 7 x $10^3$ cells were seeded on a 96-well plate (manufactured by Falcon Co., Ltd.) and compared with the control oligonucleotide-transfected sample by Cell Death Detection ELISA (Roche), using Lipofectamine 2000 (manufactured by Invitrogen Corp.) as described above, on day 3 after the antisense oligonucleotide transfection.

Consequently, the expression of EZH2 (RNA) decreased to 63% in 7 hours after the antisense oligonucleotide transfection, as compared to the expression when the control oligonucleotide was transfected (100%) and on day 3 after the transfection, apoptosis increased to 174%, as compared to the control (100%).

These results reveal that EZH2 was not only overexpressed in colon cancer, breast cancer, lung cancer, pancreatic cancer, ovary cancer, etc., but also involved in proliferation of cancer cells such as the cell line MDA-MB-231 or apoptosis.

EXAMPLE 4

The expression level of EZH2 was compared in various cancer cell lines. The cell lines shown in Table 4 were used. After incubation at 37°C, RNA was extracted in accordance with the protocol of RNeasy Mini Kit (manufactured by Qiagen, Inc.) and cDNA was prepared using TaqMan Reverse Transcription Reagents (manufactured by Applied Biosystems, Inc.). Quantitative PCR was performed on ABI PRISM™ 7900HT Sequence Detector (manufactured by Applied Biosystems, Inc.), using two primers (SEQ ID NO: 6 and SEQ ID NO: 7) and a probe (SEQ ID NO: 8), as in EXAMPLE 2. To correct the expression levels between the cells, the amount of β-actin RNA was quantified using human ACTB TaqMan MGB probe (manufactured by Applied Biosystems, Inc.).

The results revealed that the expression of EZH2 was low in normal mammary epithelial cells HMEC or MCF10A deemed relatively close to normal cells, whereas EZH2 was overexpressed in hormone-independent breast cancer cell line MDA-MB-231 or various types of lung cancer (e.g., NCI-H1299, etc.), colon cancer (e.g., RKO, etc.).

The combined results of EXAMPLE 3 indicate that EZH2 was expressed over a wide range of hormone-independent cancer, suggesting that various cancer cells causing apoptosis by suppressing the function of EZH2 would be present.

Table 4

| Template | Ratio of Gene Expression Level ( x $10^{-5}$) |
|---|---|
| HMEC | 9.1 |
| MCF10A | 3.3 |
| MDA-MB-435s | 29.3 |
| MDA-MB-231 | 66.3 |
| SKBR3 | 34.8 |
| HCC1937 | 25.5 |
| LS180 | 56.4 |
| HCT116 | 130.1 |
| HCT-15 | 198.1 |
| RKO | 114.7 |
| H1299 | 111.5 |
| A549 | 28.3 |

Table 4 (continued)

| Template | Ratio of Gene Expression Level ( x $10^{-5}$) |
|---|---|
| PANC1 | 25.8 |
| THP-1 | 25.2 |
| U937 | 28.0 |
| HL-60 | 72.9 |
| Jarkat | 86.4 |

In the table, the ratio of gene expression level represents the numerical value calculated by the following equation.

Ratio in gene expression level

= (number of EZH2 copies/μl of cancer cell-derived cDNA)/(number of β-actin

copies/μl of cancer cell-derived cDNA)

EXAMPLE 5

Since the overexpression of EZH2 in colon cancer or lung cancer became clear in EXAMPLE 4, it was examined if apoptosis was induced when the function of EZH2 was suppressed.

RNA Duplexes for the base sequence represented by SEQ ID NO: 3 (hereinafter briefly referred to as siRNA) were designed (SEQ ID NO: 11 and SEQ ID NO: 12) and provided for the transfection experiment using the synthetic duplexes (Qiagen, Inc.). For control siRNAs, commercially available Control (non-silencing) siRNAs (SEQ ID NO: 13 and SEQ ID NO: 14) (manufactured by Qiagen, Inc.) were used.

Colon cell line HCT116 (purchased from ATCC) and lung cancer cell line NCI-H1299 (purchased from ATCC) were used as the test cells used for the following test. Forty-eight hours before transfection of siRNAs, 5 x $10^5$ cells were plated on a 24-well plate (manufactured by Falcon Co., Ltd.). In transfection of these siRNAs, Lipofectamine 2000 (manufactured by Invitrogen Corp.) was used in accordance with its protocol. The final concentration of siRNAs was set at 45 nM. RNA was extracted 36 hours after the transfection, following the protocol of RNeasy Mini Kit (manufactured by Qiagen, Inc.) and cDNA was prepared using TaqMan Reverse Transcription Reagents (manufactured by Applied Biosystems, Inc.). Quantitative PCR was performed on ABI PRISM™ 7900HT Sequence Detector (manufactured by Applied Biosystems, Inc.), using two primers (SEQ ID NO: 6 and SEQ ID NO: 7) and a probe (SEQ ID NO: 8), as in EXAMPLE 4.

Meanwhile, 1 x $10^3$ cells were seeded on a 96-well plate (manufactured by Falcon Co., Ltd.) to assess the effect on apoptosis. Forty-eight hours later, siRNA or control siRNA to EZH2 was transfected using Lipofectamine 2000 (manufactured by Invitrogen Corp.) as described above. On day 3 after the siRNA transfection, the caspase 3/7 activity as an indicator of apoptosis was compared by the Caspase Glo-3/7 assay (manufactured by Promega Corp.).

Consequently, the expression of EZH2 (RNA) decreased to 13% with NCI-H1299 and to 50% with HCT116 in 36 hours after the transfection of siRNA to EZH2, as compared to the expression when control siRNA was transfected (100%). On day 3 after the transfection, apoptosis increased to 143% with NCI-H1299 and to 185% with HCT116, as compared to the control (100%).

These results reveal that EZH2 was not only overexpressed in colon cancer, breast cancer, lung cancer, pancreatic cancer, ovary cancer, etc., but also involved in proliferation of various cancer cells or apoptosis.

EXAMPLE 6

(1) Preparation of recombinant EZH2 protein

EZH2 protein is prepared to acquire a compound of inhibiting the enzyme of EZH2 protein.

PCR is carried out using the full length EZH2 gene obtained in EXAMPLE 2, or the EZH2 gene is excised with restriction enzymes appropriately chosen to insert into a vector with an appropriate tag. As the vector, for example, FLAG-tagged pFLAGCMV4 (SIGMA), etc. is used when cells are expressed in mammals. The cells are transfected in the cell line such as COS7 using Fugene6 (Roche Corp.), etc. and subjected to protein purification 2 or 3 days after. Using NE-PER kit (Pierce, Inc.), etc., the nuclear fraction is obtained and purified on M2-Agarose and FLAG peptide (both by SIGMA) to obtain the FLAG-fused EZH2 protein.

(2) Enzyme reaction

After 50 μl of reaction buffer [50mM Tris-hydrochloride (pH 8.5), 20 mM potassium chloride, 10 mM magnesium chloride, 250 mM sucrose and 10 mM 2-mercaptoethanol] containing the recombinant EZH2 protein obtained in (1) above and 2 μl of DMF solution containing a test compound are mixed on a streptoavidin-coated 96-well plate (manufactured by Perkin-Elmer) and the mixture is settled at 37°C for 10 minutes. Next, 1 ng of biotinylated histone H3 peptide (manufactured by Upstate) and [$^3$H]S-adenosylmethionine (manufactured by Amersham) (0.5μCi) are added to the system and mixed with each other to initiate the reaction. After reacting at 37°C for 3 hours, the reaction solution is discarded and washed 3 times with wash buffer [phosphate buffer containing 0.05% Tween-20], 100 μl of liquid scintillator (manufactured by Wako Pure Chemical Industries, Ltd.) is added thereto. The radioactivity is determined with a liquid scintillation counter (manufactured by Wallac). The activity obtained when a DMF solution containing no test compound is made 100 and the 50% inhibitory concentration ($IC_{50}$) is calculated. A compound, which gives a lower $IC_{50}$ value, is selected as a compound that inhibits the activity of EZH2 protein more strongly.

INDUSTRIAL APPLICABILITY

The protein of the present invention is a diagnostic marker for cancer. The compound or its salt that regulates (preferably inhibits) the activity of said protein, the compound or its salt that regulates (preferably inhibits) the expression of a gene for the protein, the antibody of the present invention, the antisense polynucleotide of the present invention can be used as a low toxic and safe pharmaceutical such as a prophylactic/therapeutic agent for a cancer (e.g., colon cancer, breast cancer, lung cancer, prostate cancer, esophageal cancer, gastric cancer, hepatic cancer, biliary tract cancer, spleen cancer, renal cancer, bladder cancer, uterine cancer, testicular cancer, thyroid cancer, pancreatic cancer, ovary cancer, brain tumor, blood tumor, etc.), preferably a prophylactic/therapeutic agent for colon cancer, breast cancer, lung cancer, pancreatic cancer or ovary cancer, an apoptosis inducing agent, etc.

## Claims

1. A prophylactic/therapeutic agent for cancer, comprising a compound or its salt that inhibits the activity of a protein comprising the same or substantially the same amino acid sequence as the amino acid sequence represented by SEQ ID NO: 1, its partial peptide, or a salt thereof.

**2.** A prophylactic/therapeutic agent for cancer, comprising a compound or its salt that inhibits the expression of a gene for a protein comprising the same or substantially the same amino acid sequence as the amino acid sequence represented by SEQ ID NO: 1, its partial peptide, or a salt thereof.

**3.** An antisense polynucleotide comprising the entire or part of a base sequence complementary or substantially complementary to a base sequence of a polynucleotide encoding a protein having the same or substantially the same amino acid sequence as the amino acid sequence represented by SEQ ID NO: 1, or its partial peptide.

**4.** A prophylactic/therapeutic agent for cancer, comprising the antisense polynucleotide according to claim 3.

**5.** A prophylactic/therapeutic agent for cancer, comprising an antibody against a protein comprising the same or substantially the same amino acid sequence as the amino acid sequence represented by SEQ ID NO: 1, its partial peptide, or a salt thereof.

**6.** The prophylactic/therapeutic agent for cancer according to claim 1, 2, 4 or 5, wherein said cancer is colon cancer, breast cancer, lung cancer, pancreatic cancer or ovary cancer.

**7.** A diagnostic agent for cancer comprising an antibody against a protein comprising the same or substantially the same amino acid sequence as the amino acid sequence represented by SEQ ID NO: 1, its partial peptide, or a salt thereof.

**8.** A diagnostic agent for cancer comprising a polynucleotide encoding a protein having the same or substantially the same amino acid sequence as the amino acid sequence represented by SEQ ID NO: 1, or its partial peptide.

**9.** The diagnostic agent according to claim 7 or 8, wherein said cancer is colon cancer, breast cancer, lung cancer, pancreatic cancer or ovary cancer.

**10.** A prophylactic/therapeutic agent for a compound or its salt having an action of inhibiting enzyme activity to transfer the methyl group(s) to the lysine 9 and/or 27 residue of histone H3.

**11.** An apoptosis inducing agent comprising a compound or its salt having an action of inhibiting enzyme activity to transfer the methyl group(s) to the lysine 9 and/or 27 residue of histone H3.

**12.** A prophylactic/therapeutic agent for cancer comprising a compound or its salt having an action of inhibiting expression of enzyme to transfer the methyl group(s) to the lysine 9 and/or 27 residue of histone H3.

**13.** An apoptosis inducing agent comprising a compound or its salt having an action of inhibiting expression of enzyme to transfer the methyl group(s) to the lysine 9 and/or 27 residue of histone H3.

**14.** A method of screening a prophylactic/therapeutic agent for cancer, which comprises using a protein comprising the same or substantially the same amino acid sequence as the amino acid sequence represented by SEQ ID NO: 1, its partial peptide, or a salt thereof.

**15.** A kit for screening a prophylactic/therapeutic agent for cancer, comprising a protein comprising the same or substantially the same amino acid sequence as the amino acid sequence represented by SEQ ID NO: 1, its partial peptide, or a salt thereof.

**16.** A method of screening a prophylactic/therapeutic agent for cancer, which comprises using a polynucleotide encoding a protein having the same or substantially the same amino acid sequence as the amino acid sequence represented by SEQ ID NO: 1, or its partial peptide.

**17.** A kit for screening a prophylactic/therapeutic agent for cancer, comprising a polynucleotide encoding a protein having the same or substantially the same amino acid sequence as the amino acid sequence represented by SEQ ID NO: 1, or its partial peptide.

**18.** An apoptosis inducing agent comprising a compound or its salt that inhibits the activity of a protein comprising the same or substantially the same amino acid sequence as the amino acid sequence represented by SEQ ID NO: 1, its partial peptide, or a salt thereof.

**19.** An apoptosis inducing agent comprising a compound or its salt that inhibits the expression of a gene for a protein comprising the same or substantially the same amino acid sequence as the amino acid sequence represented by SEQ ID NO: 1, its partial peptide, or a salt thereof.

**20.** A method of screening an apoptosis inducing agent, which comprises using a protein comprising the same or substantially the same amino acid sequence as the amino acid sequence represented by SEQ ID NO: 1, its partial peptide, or a salt thereof.

**21.** A method of screening an apoptosis inducing agent, which comprises using DNA encoding a protein having the same or substantially the same amino acid sequence as the amino acid sequence represented by SEQ ID NO: 1, or its partial peptide.

**22.** A method of preventing/treating cancer, which comprises administering to a mammal an effective dose of (i) a compound or its salt that inhibits the activity of a protein having the same or substantially the same amino acid sequence as the amino acid sequence represented by SEQ ID NO: 1, its partial peptide, or a salt thereof, (ii) a compound or its salt that inhibits the expression of a gene for said protein, its partial peptide or a salt thereof, (iii) an antibody against said protein, its partial peptide or a salt thereof, or (iv) an antisense polynucleotide comprising the entire or part of a base sequence complementary or substantially complementary to a base sequence of a polynucleotide encoding said protein or its partial peptide.

**23.** A method of inducing apoptosis, which comprises administering to a mammal an effective dose of (i) a compound or its salt that inhibits the activity of a protein having the same or substantially the same amino acid sequence as the amino acid sequence represented by SEQ ID NO: 1, its partial peptide, or a salt thereof, (ii) a compound or its salt that inhibits the expression of a gene for said protein, its partial peptide or a salt thereof, (iii) an antibody against said protein, its partial peptide or a salt thereof, or (iv) an antisense polynucleotide comprising the entire or part of a base sequence complementary or substantially complementary to a base sequence of a polynucleotide encoding said protein or its partial peptide.

**24.** A method of preventing/treating cancer, which comprises inhibiting the activity of a protein having the same or substantially the same amino acid sequence as the amino acid sequence represented by SEQ ID NO: 1, its partial peptide, or a salt thereof, or inhibiting the expression of a gene for said protein, its partial peptide, or a salt thereof.

**25.** A method of inducing apoptosis, which comprises inhibiting the activity of a protein having the same or substantially the same amino acid sequence as the amino acid sequence represented by SEQ ID NO: 1, its partial peptide, or a salt thereof, or inhibiting the expression of a gene for said protein, its partial peptide, or a salt thereof.

**26.** Use of (i) a compound or its salt that inhibits the activity of a protein having the same or substantially the same amino acid sequence as the amino acid sequence represented by SEQ ID NO: 1, its partial peptide, or a salt thereof, (ii) a compound or its salt that inhibits the expression of a gene for said protein, its partial peptide or a salt thereof, (iii) an antibody against said protein, its partial peptide or a salt thereof, or (iv) an antisense polynucleotide comprising the entire or part of a base sequence complementary or substantially complementary to a base sequence of a polynucleotide encoding said protein or its partial peptide, to manufacture a prophylactic/therapeutic agent for cancer.

**27.** Use of (i) a compound or its salt that inhibits the activity of a protein having the same or substantially the same amino acid sequence as the amino acid sequence represented by SEQ ID NO: 1, its partial peptide, or a salt thereof, (ii) a compound or its salt that inhibits the expression of a gene for said protein, its partial peptide or a salt thereof, (iii) an antibody against said protein, its partial peptide or a salt thereof, or (iv) an antisense polynucleotide comprising the entire or part of a base sequence complementary or substantially complementary to a base sequence of a polynucleotide encoding said protein or its partial peptide, to manufacture an apoptosis inducing agent.

## INTERNATIONAL SEARCH REPORT

International application No.
PCT/JP03/16417

A. CLASSIFICATION OF SUBJECT MATTER
Int.Cl$^7$ C12N15/11, C12Q1/68, A61K39/395, A61K48/00, A61P1/00, A61P11/00, A61P15/00, A61P35/00, G01N33/15, G01N33/50, G01N33/574

According to International Patent Classification (IPC) or to both national classification and IPC

B. FIELDS SEARCHED

Minimum documentation searched (classification system followed by classification symbols)
Int.Cl$^7$ C12N15/11, C12Q1/68

Documentation searched other than minimum documentation to the extent that such documents are included in the fields searched

Electronic data base consulted during the international search (name of data base and, where practicable, search terms used)
SwissProt/PIR/GeneSeq, Genbank/EMBL/DDBJ/GeneSeq, WPI(STN), BIOSIS(STN), MEDLINE(STN), CA(STN)

C. DOCUMENTS CONSIDERED TO BE RELEVANT

| Category* | Citation of document, with indication, where appropriate, of the relevant passages | Relevant to claim No. |
|---|---|---|
| X | Sooryanarayana V. et al., The polycomb group protein EZH2 is involved in progression of prostate cancer, Nature 2002, Vol.419, pages 624 to 629 | 2-5,7,8,12, 14-17,26 |
| X | WO 02/86443 A2 (EOS BIOTECHNOLOGY, INC.), 31 October, 2002 (31.10.02), P232, SEQ ID NO: 113 (Family: none) | 2-9,12, 14-17,26 |
| X | JP 2001-505402 A (BOEHRINGER INGELHEIM INT.), 24 April, 2001 (24.04.01), & WO 96/35784 A2 & EP 827537 A2 | 2-5,7,8,12, 14-17,26 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 February, 2004 (25.02.04) | 09 March, 2004 (09.03.04) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/16417

C (Continuation). DOCUMENTS CONSIDERED TO BE RELEVANT

| Category* | Citation of document, with indication, where appropriate, of the relevant passages | Relevant to claim No. |
|---|---|---|
| P,X | WO 03/12067 A2 (UNIV MICHIGAN), 13 February, 2003 (13.02.03), P88 SEQ ID NO:95 & US 2003/175736 A1 | 2-5,7,8,12, 14-17,26 |
| P,X | WO 03/70887 A2 (RIBOZYME PHARM. INC.), 28 August, 2003 (28.08.03), (Family: none) | 2-5,7,8,12, 14-17,26 |
| A | WO 01/94629 A2 (AVALON PHARMASEUTICALS), 13 December, 2001 (13.12.01), SEQ ID NO: 4184 & US 2002/150877 A1 | 2-9,12-17, 19-21,26,27 |
| A | Cao R., Wang L. et al., Role of histone H3 lysine 27 methylation in Polycomb-group silencing., Science 2002, Vol.298, pages 1039 to 1043 | 2-9,12-17, 19-21,26,27 |

Form PCT/ISA/210 (continuation of second sheet) (July 1998)

**INTERNATIONAL SEARCH REPORT**

International application No.
PCT/JP03/16417

**Box I Observations where certain claims were found unsearchable (Continuation of item 2 of first sheet)**

This international search report has not been established in respect of certain claims under Article 17(2)(a) for the following reasons:

1. [X] Claims Nos.: 22, 23, 24, 25
because they relate to subject matter not required to be searched by this Authority, namely:
Claims 22 to 25 pertain to methods for treatment of the human body by therapy and thus relate to a subject matter which this International Searching Authority is not required, under the provisions of Article 17(2)(a)(i) of the PCT and Rule 39.1(v) of the Regulations under the PCT, to search.

2. [X] Claims Nos.: 1, 2, 6, 10-13, 18, 19, 26, 27
because they relate to parts of the international application that do not comply with the prescribed requirements to such an extent that no meaningful international search can be carried out, specifically:
The compound as set forth in claims 1, 6, 10, 11, 18, 16, 17, 26 and 27 relates to a compound inhibiting the activity of the protein as defined in claim 1 and a composition containing a compound defined by a desired property. Although (continued to extra sheet)

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP03/16417

Continuation of Box No. I-2 of continuation of first sheet(1)

the above compound involves any compounds having such a property, the description presents no specific example of the above compound. Thus, these claims are neither disclosed in the meaning within PCT Article 5 nor supported by the description in the meaning within PCT Article 6. Even though the common technical knowledge at the point of the application is taken into consideration, it is completely unknown what specific compounds are involved and what are not. Thus, these claims are extremely unclear and do not comply with the requirement of clearness in accordance with PCT Article 6 too.

Such being the case, no meaningful search can be made on the inventions according to the above claims.

Claims 2, 6, 12, 13, 19, 26 and 27 relate to a composition containing a compound which is defined by a desired property of inhibiting the expression of a gene of the protein as defined in claim 1. Although the above compound involves any compounds having such a property, nothing but the compound as specified in claim 3 is disclosed in the meaning within PCT Article 5. and supported by the description in the meaning within PCT Article 6. Thus, it appears that these claims are not supported by the disclosure of the description in the meaning within PCT Article 6.

Even though the common technical knowledge at the point of the application is taken into consideration, the scope of the compound having such a property cannot be specified. Thus, claims 2, 6, 12, 13, 19, 26 and 27 do not comply with the requirement of clearness in accordance with PCT Article 6 too.

Such being the case, the search was made exclusively on a composition containing the compound as specified in claim 3.

Form PCT/ISA/210 (extra sheet) (July 1998)